# EUROPEAN PATENT SPECIFICATION

(11) **EP 3 377 478 B1**
(45) Date of publication and mention of the grant of the patent: **24.07.2019**
(21) Application number: 16797941.8
(22) Date of filing: 18.11.2016
(51) Int. Cl.: C07D 401/04, A61K 31/445, A61P 37/08

(54) **CO-CRYSTALS OF BILASTINE**
COKRISTALLE VON BILASTINE
CO-CRISTAUX DE BILASTINE

(30) Priority: 20.11.2015 EP 15382577
(43) Date of publication of application: 26.09.2018
(73) Proprietor: FAES FARMA, S.A., 48940 Leioa - Vizcaya (ES)
(72) Inventor: HERNÁNDEZ HERRERO, Gonzalo, E-48940 Leioa (Vizcaya) (ES); RUBIO ROYO, Víctor, E-48940 Leioa (Vizcaya) (ES); GARCÍA DOMÍNGUEZ, Neftalí, E-48940 Leioa (Vizcaya) (ES); CANAL MORI, Gonzalo, E-48940 Leioa (Vizcaya) (ES); TESSON, Nicolas, E-08028 Barcelona (ES); JIMÉNEZ GONZÁLEZ, Carme, E-08028 Barcelona (ES); DE MIER VINUE, Jordi, E-08028 Barcelona (ES)
(74) Representative: ABG Intellectual Property, S.L.
(86) International application number: PCT/EP2016/078145
(87) International publication number: WO 2017/085258

(56) References cited:
- WO-A1-03/089425
- WO-A2-2008/054609
- CN-A- 103 351 380
- RICHARD J BASTIN ET AL: "Salt Selection and Optimisation Procedures for Pharmaceutical New Chemical Entities", ORGANIC PROCESS RESEARCH AND DEVELOPMENT, AMERICAN CHEMICAL SOCIETY, US, vol. 4, no. 5, 19 July 2000 (2000-07-19), pages 427-435, XP008154792, ISSN: 1083-6160, DOI: 10.1021/OP000018U [retrieved on 2000-07-19]
- BERGE S M ET AL: "PHARMACEUTICALS SALTS", JOURNAL OF PHARMACEUTICAL SCIENCES, AMERICAN PHARMACEUTICAL ASSOCIATION, WASHINGTON, US, vol. 66, no. 1, 1 January 1977 (1977-01-01), pages 1-19, XP000562636, ISSN: 0022-3549, DOI: 10.1002/JPS.2600660104

## Description

### FIELD OF THE INVENTION

The present invention relates to cocrystals of benzimidazole compounds, process for obtaining thereof and their use in pharmaceutical compositions, particularly their use for antihistamine and antiallergic compositions. The invention also relates to a method for increasing the aqueous solubility of benzimidazole compounds.

### BACKGROUND

It has long been known that histamine plays a very important role in allergic-type diseases, such as allergic rhinitis, conjunctivitis, rhinoconjunctivitis, dermatitis, urticaria and asthma. Antihistaminic compounds acting at the H₁-receptor histamine level are useful for treating such conditions.

Documents EP 0818454 A1 and EP 0580541 A1 disclose benzimidazole compounds with selective H₁ antihistaminic activity and devoid of arrhythmogenic effects. Patent application EP14382576.8 also discloses benzimidazole compounds having potent selective H₁ antihistaminic activity, lacking activity on the central nervous system and on the cardiovascular system.

2-[4-(2-{4-[1-(2-Ethoxyethyl)-1H-benzimidazol-2-yl]-1-piperidinyl}ethyl)phenyl]-2-methylpropanoic acid, also known as bilastine, having formula: 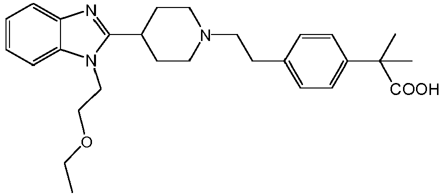 and developed by Faes Farma, Spain, is a H₁ antagonist benzimidazole compound with no sedative side effects, no cardiotoxic effects, and no hepatic metabolism. In addition, bilastine has proved to be effective for the symptomatic treatment of allergic rhinoconjunctivitis and urticaria.

The above benzimidazole compounds with selective H₁ antihistaminic activity present low solubility in water, which impedes the development of pharmaceutically acceptable means of administering said compounds in liquid form. For example, the solubility of bilastine in the pH range 5-8 is around 500 µg/mL.

Crystalline forms of bilastine are described in the art. Forms I, II and III are disclosed in WO 2003/089425 A1, and a further polymorph has been described in CN103788062A. Bilastine dihydrate crystalline forms are disclosed in SK50032014 U1 and SK7066 Y1.

Therefore, there is a need in the art to provide a method for improving the solubility in water of said benzimidazole compounds with selective H₁ antihistaminic activity. Particularly, there is a need to improve the solubility of the mentioned compounds at the physiologic pH of the eye (6.5-8.0) and the nasal cavity (5.5-8.3) and also for compositions for parenteral administration (pH 3.0-10.5), especially in the physiologic pH of the blood (pH 7.35-7.45). Therefore, the development of soluble and stable pharmaceutically acceptable forms of said compounds, and particularly of bilastine, that can be obtained robustly and reproducibly using scalable crystallization procedures is highly desirable. The present invention addresses such concerns.

### BRIEF DESCRIPTION OF THE INVENTION

The applicant of the present invention has surprisingly found that bilastine forms cocrystals with certain organic molecules. These cocrystals are stable, most of them are stable even under forced conditions of temperature and humidity, thus improving their handling and storage and so the production of pharmaceutical compositions comprising them. Besides, these cocrystals enhance the solubility of bilastine thus improving the production and use of pharmaceutical compositions for being administrated for instance in the eye, the nasal cavity, and for parenteral administration.

Therefore, one aspect of the present invention relates to a cocrystal comprising:
a) at least bilastine; and
b) at least a cocrystal forming compound selected from the group consisting of glutaric acid, adipic acid, sorbic acid, succinic acid, benzoic acid, 4-aminobenzoic acid, L-malic acid, resorcinol, methyl 4-hydroxy benzoate and N-(4-hydroxyphenyl)acetamide and mixtures thereof,
or a solvate thereof.

Another aspect of this invention refers to a process for the preparation of a cocrystal of the invention comprising:
a) stirring a mixture of bilastine, or a pharmaceutically acceptable solvate or polymorph thereof, and the neutral cocrystal forming compound in an appropriate solvent between room temperature and 40°C;
b) cooling the mixture to room temperature if temperature of step a) is higher than room temperature, and
c) isolating the obtained compound.

Another further aspect of this invention refers to a process for the preparation of a cocrystal of the invention comprising:
a) wet grinding of bilastine, or a pharmaceutically acceptable solvate or polymorph thereof, and the neutral cocrystal forming compound in an appropriate solvent, and
b) isolating the obtained compound.

Another aspect of this invention refers to a pharmaceutical composition comprising a cocrystal of the invention, and a pharmaceutically acceptable excipient.

An additional aspect of this invention refers to a cocrystal of the present invention for use as a medicament.

Another aspect refers to a cocrystal of the present invention for use in the treatment and/or prevention of a disorder or disease susceptible to amelioration by antagonism of H1 histamine receptor such as allergic disorders or diseases.

### BRIEF DESCRIPTION OF THE FIGURES

**Fig. 1****.** Characterization of bilastine BLN(I) - glutaric acid cocrystals: Fig. 1.1.a) XRPD of GL(I); Fig. 1.1.b) ¹H-RMN of GL(I) (in DMSO); Fig. 1.1.c) TGA of GL(I); Fig. 1.1.d) Solubility comparison BLN(I) vs GL(I). Fig. 1.2.a) XRPD of GL(IV); Fig. 1.2.b) ¹H-RMN of GL(IV) (in DMSO); Fig. 1.2.c) TGA of GL(IV); Fig. 1.2.d) Solubility comparison BLN(I) vs GL(IV). Fig. 1.3.a) XRPD of GL(V); Fig. 1.3.b) ¹H-RMN of GL(V) (in DMSO); Fig. 1.3.c) TGA of GL(V); Fig. 1.3 d) Solubility comparison BLN(I) vs GL(V).
**Fig. 2****.** Characterization of bilastine BLN(I) and adipic acid cocrystals: Fig. 2.1.a) XRPD of AD (I); Fig. 2.1.b) ¹H-RMN of AD (I) (in DMSO); Fig. 2.1.c) TGA of AD(I); Fig. 2.1.d) Solubility comparison BLN(I) vs AD(I). Fig. 2.2.a) XRPD of AD (III); Fig.2.2.b) ¹H-RMN of AD (III) (in DMSO); Fig. 2.2.c) TGA of AD(III); Fig. 2.2.d) Solubility comparison BLN(I) vs AD(III).
**Fig. 3****.** Characterization of bilastine BLN(I) and sorbic acid cocrystal: Fig. 3.1.a) XRPD of SO (I); Fig.3.1.b) ¹H-RMN of SO (I) (in DMSO); Fig.3.1.c) TGA of SO(I); Fig. 3.1.d) Solubility comparison BLN(I) vs SO(I).
**Fig. 4****.** Characterization of bilastine BLN(I) and succinic acid cocrystals: Fig. 4.1.a) XRPD of SU(VI); Fig. 4.1.b) ¹H-RMN of SU(VI) (in DMSO); Fig. 4.1.c) TGA of SU(VI); Fig. 4.1.d) Solubility comparison BLN(I) vs SU(VI). Fig. 4.2.a) XRPD of SU(VII); Fig.4.2.b) ¹H-RMN of SU(VII) (in DMSO); Fig. 4.2.c) TGA of SU(VII); Fig. 4.2.d) Solubility comparison BLN(I) vs SU(VII).
**Fig. 5****.** Characterization of bilastine BLN(I) and benzoic acid cocrystals: Fig. 5.1.a) XRPD of BE(I); Fig. 5.1.b) ¹H-RMN of BE(I) (in DMSO); Fig. 5.1.c) TGA of BE(I); Fig. 5.1.d) Solubility comparison BLN(I) vs BE(I). Fig. 5.2.a) XRPD of BE(II); Fig.5.2.b) ¹H-RMN of BE(II) (in DMSO); Fig. 5.2.c) TGA of BE(II); Fig. 5.2.d) Solubility comparison BLN(I) vs BE(II).
**Fig. 6****.** Characterization of bilastine BLN(I) and 4-aminobenzoic acid cocrystals: Fig. 6.1.a) XRPD of AB(VII); Fig. 6.1.b) ¹H-RMN of AB(VII) (in DMSO); Fig. 6.1.c) TGA of AB(VII); Fig. 6.1.d) Solubility comparison BLN(I) vs AB(VII). Fig. 6.2.a) XRPD of AB(VIII); Fig.6.2.b) ¹H-RMN of AB(VIII) (in DMSO); Fig. 6.2.c) TGA of AB(VIII); Fig. 6.2.d) Solubility comparison BLN(I) vs AB(VIII).
**Fig. 7****.** Characterization of bilastine BLN(I) and L-malic acid cocrystal: Fig. 7.1.a) XRPD of L-ML(I); Fig.7.1.b) ¹H-RMN of L-ML(I) (in DMSO); Fig.7.1.c) TGA of L-ML(I); Fig. 7.1.d) Solubility comparison BLN(I) vs L-ML(I).
**Fig. 8****.** Characterization of bilastine BLN(I) and resorcinol cocrystal: Fig. 8.1.a) XRPD of RE(IV); Fig.8.1.b) ¹H-RMN of RE(IV) (in DMSO); Fig.8.1.c) TGA of RE(IV); Fig. 8.1.d) Solubility comparison BLN(I) vs RE(IV).
**Fig. 9****.** Characterization of bilastine BLN(I) and methyl 4-hydroxybenzoate cocrystal: Fig. 9.1.a) XRPD; Fig.9.1.b) ¹H-RMN; Fig.9.1.c) TGA; Fig. 9.1.d) FT-IR.
**Fig. 10****.** Characterization of bilastine BLN(I) and N-4-hydroxyphenylacetamide cocrystal: Fig. 10.1.a) XRPD; Fig.10.1.b) FT-IR.

### DETAILED DESCRIPTION OF THE INVENTION

In the context of the present invention, the following terms have the meaning detailed below.

The term "alkyl" refers to a linear or branched saturated hydrocarbon chain radical consisting of carbon and hydrogen atoms and which is attached to the rest of the molecule by a single bond. Alkyl groups include for example and in a non-limiting sense, methyl, ethyl, n-propyl, i-propyl, n-butyl, t-butyl, n-pentyl, etc. Preferably "alkyl" refers to methyl or ethyl.

As understood in this technical area, there can be a certain degree of substitution on the previously defined radicals. The references to substituted groups indicate that the specified radical can be substituted in one or more available positions by one or more substituents.

### Bilastine

The cocrystal of the invention comprises bilastine of formula,

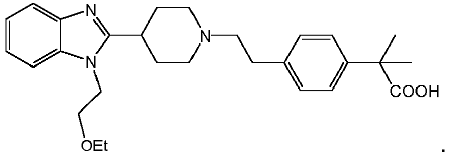

This compound is 2-[4-(2-{4-[1-(2-ethoxyethyl)-1H-benzimidazol-2-yl]-1-piperidinyl}ethyl)phenyl]-2-methylpropanoic acid, also known as bilastine. The synthesis of bilastine has been described in EP 0818454 A1.

### Cocrystal

The term "cocrystal" or "co-crystal" refers herein to a crystalline entity with at least two different components or molecules constituting the unit cell at room temperature (20-25 °C) and interacting by weak interactions. Thus, in a cocrystal a first neutral component crystallizes with at least one second neutral component, and interact via non-ionic interactions. Said at least one second component in the cocrystal is commonly referred to as a "cocrystal former" or "coformer" and is solid at room temperature and atmospheric pressure. This definition distinguishes cocrystals from crystalline solvates, in that in a solvate one of the components is a liquid at room temperature and atmospheric pressure. However, the cocrystals of the invention may further include one or more solvent molecules in the crystal lattice, that is, this invention also includes solvates of the cocrystals. Besides, it should be noted that, unlike salts, where the components in the crystal lattice are in an ionized state, the cocrystal's components are in a neutral state and are linked by hydrogen bonding and other non-ionic interactions. Cocrystals are also defined in this invention (according to the FDA's guideline for the classification of cocrystals) as having a pKa difference between the at least two cocrystal components lower than 1, thus indicating a non-ionic species (that is, they are not salts) and minimal proton sharing.

The cocrystals of the present invention comprise at least the first neutral component bilastine and a second neutral cocrystal forming compound. Without wishing to be bound by any particular theory, it is believed that the first neutral component bilastine can either exist as a neutral zwitterionic species, wherein the acidic moiety is deprotonated and, simultaneously, the pyridinic nitrogen of the benzimidazole moiety is protonated or, as a neutral species, in which both acidic and benzimidazole moieties are neutral. In either case, the net charge of bilastine is zero and thus the first component of the cocrystal is indeed neutral. The second neutral cocrystal forming component is believed to lack any charges due to the pKa difference between the two cocrystal components and therefore it does not form ionic interactions with other molecules. In this way, it is believed that the cocrystal of the present invention comprises a first neutral bilastine component and a second neutral cocrystal forming compound that are linked by hydrogen bonding and other non-ionic interactions. The cocrystal of the present invention is not a salt.

A "pharmaceutically acceptable cocrystal" is understood to be a cocrystal wherein one of the components constituting the unit cell is an active pharmaceutical ingredient (API). Thus, in a preferred embodiment, this invention describes a pharmaceutically acceptable cocrystal wherein the API is bilastine. Further, according to the Regulatory Classification of Pharmaceutical Co-Crystals (FDA, April 2013), a pharmaceutically acceptable cocrystal of bilastine in the present invention presents a ΔpKa bilastine-coformer of less than 1. In this particular case, as bilastine is a zwitterion, the base function is a protonated amine (pKa 8.8) and the acid function is the carboxylate (pKa 4.4).

### Coformer

The cocrystal of the invention comprises at least a neutral cocrystal forming compound selected from an organic molecule, which is the coformer.

In a particular embodiment said organic molecule is selected from the group consisting of carboxylic acids and phenolic compounds.

Further, according to the definition of cocrystal the coformers of the present invention are solid at room temperature. In further agreement with the definition of cocrystal, the ΔpKa bilastine-coformer is less than 1.

In a particular embodiment the at least one cocrystal forming compound is an organic molecule selected from carboxylic acids. In another particular embodiment these carboxylic acids are selected from the group consisting of substituted or unsubstituted aliphatic C₃-C₈ α,ω-dicarboxylic acids and mixtures thereof wherein the substituent is selected from -OH and -NH₂ groups; substituted or unsubstituted aliphatic C₃-C₆ monocarboxylic acids and mixtures thereof wherein the substituent is selected from - OH and -NH₂ groups; and substituted or unsubstituted benzoic acid and mixtures thereof, wherein the substituent is selected from -OH and -NH₂ groups. It should be noted that a benzoic acid substituted with an -OH group, although is also a phenol function, the compound is considered in this invention as a carboxylic acid.

In the following table are shown, as an example, the ΔpKa bilastine-coformer of possible coformers:

| Coformers | ΔpKa |
|---|---|
| Adipic acid | -0.04 |
| L-Malic acid | 0.94 |
| benzoic acid | 0.21 |
| succinic acid | 0.19 |
| glutaric acid | 0.06 |
| 4-Aminobenzoic acid | -0.20 |
| sorbic acid | -0.40 |

"Aliphatic C₃-C₈ α,ω-dicarboxylic acid" refers to an organic compound containing two carboxyl functional groups at the two ends of a C₃-C₈ saturated or unsaturated aliphatic chain, preferably a C₃-C₆ saturated or unsaturated aliphatic chain ("aliphatic C₃-C₆ α,ω-dicarboxylic acid"). That is, it is a compound of formula HOOC-R'-COOH, wherein R' is a saturated or unsaturated C₁-C₆, preferably a C₁-C₄, aliphatic chain. Preferably the R' group is a saturated chain.

In a particular embodiment, the R' group is an unsubstituted saturated chain, that is R' is -(CH₂)ₙ- wherein n is 1, 2, 3, 4, 5 or 6, preferably n is 1, 2, 3 or 4.

The aliphatic C₃-C₈ α,ω-dicarboxylic acids can be substituted or unsubstituted. The substituents may be selected from the group consisting of -OH, and -NH₂, preferably is -OH.

In an embodiment, the aliphatic C₃-C₈ α,ω-dicarboxylic acid is an aliphatic C₄ α,ω-dicarboxylic acid, an aliphatic C₅ α,ω-dicarboxylic acid, an aliphatic C₆ α,ω-dicarboxylic acid or an aliphatic C₇ α,ω-dicarboxylic acid and mixtures thereof.

In a particular embodiment, the aliphatic C₃-C₈ α,ω-dicarboxylic acid is selected from glutaric acid, adipic acid, sorbic acid, succinic acid and malic acid, e.g. L-malic acid and mixtures thereof. Preferably, it is glutaric acid.

"Aliphatic C₃-C₆ monocarboxylic acid" refers to an organic compound containing saturated or unsaturated aliphatic chains containing one carboxyl functional group. The carboxyl functional group is preferably present at one end of the chain, having a molecular formula of R"-COOH, wherein R" is the saturated or unsaturated C₃-C₆ aliphatic chain. In one embodiment the aliphatic chain R" is a saturated chain of between 2 and 5 carbon atoms and preferably is a saturated chain of 2, 3 or 4 carbon atoms. In another embodiment the aliphatic chain is an unsaturated chain of 3, 4 or 5 carbon atoms, preferably 5 carbon atoms. In another embodiment the aliphatic chain is a chain of 4 or 5 carbon atoms having two double bonds. In a preferred embodiment the aliphatic chain R" is an unsaturated chain having 5 carbon atoms and two double bonds, and more preferably the aliphatic monocarboxylic acid is sorbic acid. The aliphatic C₃-C₆ monocarboxylic acids can be substituted or unsubstituted. The substituent of the substituted aliphatic C₃-C₆ monocarboxylic acids is selected from the group consisting of -OH and -NH₂.

In one embodiment the carboxylic acid is unsubstituted benzoic acid. In another embodiment the carboxylic acid is substituted benzoic acid. The substituent of the substituted benzoic acid is selected from the group consisting of -OH and -NH₂, preferably -NH₂ in *para-* (*p*-) position regarding the -COOH group.

In a particular embodiment, the aliphatic C₃-C₆ monocarboxylic acid is selected from benzoic acid and 4-amino benzoic acid.

In a preferred embodiment the carboxylic acid is selected from the group consisting of glutaric acid, adipic acid, sorbic acid, succinic acid, benzoic acid, malic acid, and 4-aminobenzoic acid. In a more preferred embodiment the carboxylic acid is selected from the group consisting of glutaric acid, adipic acid, sorbic acid, succinic acid and benzoic acid. In another embodiment the carboxylic acid is selected from the group consisting of glutaric acid, adipic acid, sorbic acid and succinic acid. In a preferred embodiment the carboxylic acid is glutaric acid.

In a particular embodiment the at least one cocrystal forming compound is a phenolic compound. Phenolic compounds according to this invention refer to aromatic hydrocarbon ring(s) substituted with at least one hydroxyl group and which may be further substituted with other functional groups. In an embodiment, said other functional groups are not -COOH. In a particular invention said further functional groups can be selected from the group consisting of -OH, -NH₂, -COO-alkyl and -NH-CO-alkyl. In a preferred embodiment the further functional group can be selected from the group consisting of -OH, -NH₂, -COO-CH₃ and -NH-CO-CH₃. In a preferred embodiment the aromatic hydrocarbon ring is a benzene ring. In a particular embodiment the aromatic ring is a benzene ring and the further functional group is in para- or meta- position regarding the at least one -OH group. In a particular embodiment the phenolic compound is selected from the group consisting of resorcinol, methyl 4-hydroxybenzoate and N-(4-hydroxyphenyl)acetamide.

Further, cocrystals are capable of existing as different packing arrangements of the same molecular components to give different "polymorphic forms", "polymorphic form phase" or "crystal forms", of the same pair bilastine-coformer having the same bilastine:coformer ratio. That is, polymorphic forms may contain the components of a cocrystal of the invention, or a solvate thereof, in the same molar ratio, but presenting a different crystal structure or "crystal form". This may include solvation or hydration products.

Cocrystals of the present invention may contain the at least two components in different molar ratios. In this invention, cocrystals may contain any molar ratio of bilastine to cocrystal former but would typically be in the range of 3:1 to 1:1, and preferably the molar ratio of bilastine to cocrystal former is from 2:1 to 1:1, more preferably the molar ratio of bilastine to cocrystal former is 2:1 or 1:1. In a preferred embodiment all the above molar ratios apply to bilastine.

Additionally, cocrystals can also contain solvent molecules, e.g. water, to form cocrystal solvates or more particularly, hydrates. That is, in one embodiment the cocrystals of the present invention contain water molecules to form cocrystal hydrates. In this invention, cocrystal hydrates can contain any molar ratio of cocrystal:water but would typically be between 1:0.5 and 1:3.

### Stability

The cocrystals of this invention are stable, wherein "stable" means that the cocrystals maintain their crystalline form over various days, preferably 1, 2, 3 or 4 days or at least one night, at standard ambient conditions of temperature and pressure. In a particular embodiment the term stable means that the cocrystals maintain their crystalline form over various days: 1, 2, 3 or 4 days, or 1 week, or at least one night in forced conditions of humidity (75 ± 5% RH) and temperature (40 °C). In a particular embodiment the cocrystals of this invention maintain their crystalline form after one week under forced conditions of humidity (70 ± 5% RH) and temperature (40 °C). However, it should be considered that the hydrates may vary their water content depending of the atmospheric conditions, therefore the water content in a cocrystal hydrate may be defined within a range, as shown above.

### Particular embodiments

In one embodiment the cocrystal comprises bilastine and glutaric acid. In a particular embodiment the molar ratio of bilastine:glutaric acid is 1:1. In a particular embodiment the molar ratio of bilastine:glutaric acid is 2:1. In another particular embodiment the cocrystal of bilastine and glutaric acid contains water molecules, i.e. it is a hydrate. In an embodiment the molar ratio bilastine:glutaric acid:water in said hydrate is between 2:1:3 and 2:1:1.

In a particular embodiment this invention discloses a crystalline form, named GL(I), of a cocrystal of bilastine:glutaric acid in a 2:1 molar ratio having a X-ray powder diffraction pattern showing characteristic peaks at a reflection angle [2Θ in degrees] as disclosed in Table 1.1 ± 0.2°.

In a particular embodiment this invention discloses a crystalline form, named GL(IV), of a cocrystal of bilastine:glutaric acid in a 2:1 molar ratio having a X-ray powder diffraction pattern showing characteristic peaks at a reflection angle [2Θ in degrees] as disclosed in Table 1.2 ± 0.2°.

In a particular embodiment this invention discloses a crystalline form, named GL(V), of a cocrystal of bilastine and glutaric acid in a 1:1 molar ratio having a X-ray powder diffraction pattern showing characteristic peaks at a reflection angle [2Θ in degrees] as disclosed in Table 1.3 ± 0.2°.

In one embodiment the cocrystal comprises bilastine and adipic acid. In a particular embodiment the molar ratio of bilastine:adipic acid is 1:1. In a particular embodiment the molar ratio of bilastine:adipic acid is 2:1. In another particular embodiment the cocrystal of bilastine and adipic acid having a molar ratio of bilastine:adipic acid 2:1 is a hydrate. In an embodiment said molar ratio of bilastine:adipic acid:water in said hydrate is between 2:1:3 and 2:1:1.

In a particular embodiment this invention discloses a crystalline form, named AD(I), of a cocrystal of bilastine:adipic acid in a 1:1 molar ratio having a X-ray powder diffraction pattern showing characteristic peaks at a reflection angle [2Θ in degrees] as disclosed in Table 2.1 ± 0.2°.

In a particular embodiment this invention discloses a crystalline form, named AD(III), of a cocrystal of bilastine:adipic acid in a 2:1 molar ratio, having a X-ray powder diffraction pattern showing characteristic peaks at a reflection angle [2Θ in degrees] as disclosed in Table 2.2 ± 0.2°.

In one embodiment the cocrystal comprises bilastine and sorbic acid. In a more particular embodiment the molar ratio of bilastine:sorbic acid is 2:1. In another particular embodiment the cocrystal of bilastine and sorbic acid contains water molecules, i.e. it is a hydrate. In an embodiment the molar ratio bilastine:sorbic acid:water in said hydrate is between 2:1:4 and 2:1:1, preferably 2:1:3.8.

In a particular embodiment this invention discloses a crystalline form, named SO(I), of a cocrystal of bilastine:sorbic acid having a X-ray powder diffraction pattern showing characteristic peaks at a reflection angle [2Θ in degrees] as disclosed in Table 3.1 ± 0.2°.

In one embodiment the cocrystal comprises bilastine and succinic acid. In a particular embodiment the molar ratio of bilastine:succinic acid is 1:1. In a particular embodiment the molar ratio of bilastine:succinic acid is 2:1.

In a particular embodiment this invention discloses a crystalline form, named SU(VI), of a cocrystal of bilastine:succinic acid in a 1:1 molar ratio having a X-ray powder diffraction pattern showing characteristic peaks at a reflection angle [2Θ in degrees] as disclosed in Table 4.1 ± 0.2°.

In a particular embodiment this invention discloses a crystalline form, named SU(VII), of a cocrystal of bilastine:succinic acid in a 2:1 molar ratio having a X-ray powder diffraction pattern showing characteristic peaks at a reflection angle [2Θ in degrees] as disclosed in Table 4.2 ± 0.2°.

In one embodiment the cocrystal comprises bilastine and benzoic acid. In a particular embodiment the molar ratio of bilastine:benzoic acid is 1:2. In a particular embodiment the molar ratio of bilastine:benzoic acid is 2:1. In another particular embodiment the cocrystal of bilastine and benzoic acid contains water molecules, i.e. it is a hydrate. In an embodiment the molar ratio bilastine:benzoic acid:water in said hydrate is between 2:1:3 and 2:1:1, preferably 2:1:2.8.

In a particular embodiment this invention discloses a crystalline form, named BE(I), of a cocrystal of bilastine:benzoic acid in a 1:2 molar ratio having a X-ray powder diffraction pattern showing characteristic peaks at a reflection angle [2Θ in degrees] as disclosed in Table 5.1 ± 0.2°.

In a particular embodiment this invention discloses a crystalline form, named BE(II), of a cocrystal of bilastine:benzoic acid in a 2:1 molar ratio having a X-ray powder diffraction pattern showing characteristic peaks at a reflection angle [2Θ in degrees] as disclosed in Table 5.2 ± 0.2°.

In one embodiment the cocrystal comprises bilastine and 4-aminobenzoic acid. In a particular embodiment the molar ratio of bilastine:4-aminobenzoic acid is 1:1. In a particular embodiment the molar ratio of bilastine: 4-aminobenzoic acid is 2:1. In another embodiment the cocrystal of bilastine and 4-aminobenzoic acid contains water molecules, i.e. it is a hydrate. In an embodiment the molar ratio bilastine:4-aminobenzoic acid:water in said hydrate is between 2:1:3 and 2:1:1, preferably 2:1:2.4.

In a particular embodiment this invention discloses a crystalline form, named AB(VII), of a cocrystal of bilastine:4-aminobenzoic acid in a 1:1 molar ratio having a X-ray powder diffraction pattern showing characteristic peaks at a reflection angle [2Θ in degrees] as disclosed in Table 6.1 ± 0.2°.

In a particular embodiment this invention discloses a crystalline form, named AB(VIII), of a cocrystal of bilastine:4-aminobenzoic acid in a 2:1 molar ratio having a X-ray powder diffraction pattern showing characteristic peaks at a reflection angle [2Θ in degrees] as disclosed in Table 6.2 ± 0.2°.

In one embodiment the cocrystal comprises bilastine and malic acid, in a preferred embodiment the malic acid is L-malic acid. In a particular embodiment the molar ratio of bilastine:L-malic acid is 2:1. In a particular embodiment the cocrystal of bilastine and L-malic acid contains water molecules, i.e. it is a hydrate. In a preferred embodiment the molar ratio bilastine:L-malic acid:water in said hydrate is between 2:1:4 and 2:1:1, preferably 2:1:3.6.

In a particular embodiment this invention discloses a crystalline form, named L-ML(I), of a cocrystal of bilastine:L-malic acid in a 2:1 molar ratio having a X-ray powder diffraction pattern showing characteristic peaks at a reflection angle [2Θ in degrees] at as disclosed in Table 7.1 ± 0.2°.

In one embodiment the cocrystal comprises bilastine and resorcinol. In a particular embodiment the molar ratio of bilastine:resorcinol is 2:3. In a particular embodiment the molar ratio of bilastine:resorcinol is 2:1. In another embodiment the cocrystal of bilastine and resorcinol contains water molecules, i.e. it is a hydrate.

In a particular embodiment this invention discloses a crystalline form, named RE(IV), of a cocrystal of bilastine:resorcinol in a 2:1 molar ratio having a X-ray powder diffraction pattern showing characteristic peaks at a reflection angle [2Θ in degrees] as disclosed in Table 8.1 ± 0.2°.

In one embodiment the cocrystal comprises bilastine and methyl 4-hydroxybenzoate. In a particular embodiment the molar ratio of bilastine:methyl 4-hydroxybenzoate is 2:1. In a particular embodiment the cocrystal of bilastine and methyl 4-hydroxybenzoate contains water molecules, i.e. it is a hydrate.

In one embodiment the cocrystal comprises bilastine and N-(4-hydroxyphenil)acetamide. In a particular embodiment the cocrystal of bilastine and N-(4-hydroxyphenil)acetamide contains water molecules, i.e. it is a hydrate.

### Process

The present invention also refers to a process for preparing a cocrystal according to the invention. Two possible general processes are here disclosed for obtaining said cocrystals: slurrying and wet grinding (aka, liquid or solvent assisted grinding). In preferred embodiments the processes refer to the preparation of cocrystals of bilastine or hydrates thereof.

"Appropriate solvent" as used herein means a solvent or mixture of solvents selected from the group consisting of water, acetonitrile, dimethylsulfoxide (DMSO), methanol (MeOH), ethanol (EtOH), isopropyl alcohol (IPA), ethyl acetate (AcOEt), isobutyl acetate, (AcOⁱBu) acetone, methyl isobutyl ketone (MIK), tetrahydrofurane (THF), dioxane, diethylether, methyl tert-butyl ether (TBME), dichloromethane, chloroform, toluene, cyclohexane, xylene, heptane, dimethylformamide (DMF) and N-methyl-2-pyrrolidone (NMP), preferably water, acetonitrile, methanol, ethanol and chloroform. In a particular embodiment the solvent is water or a mixture of water and other of the above mentioned "appropriate solvents". In another embodiment the solvent is ethanol or a mixture of ethanol and other of the above mentioned "appropriate solvents".

As used herein, "room temperature" or its abbreviation "rt" is taken to mean between 20 to 25 °C. "Standard ambient conditions of temperature and pressure" or "standard ambient conditions" mean a temperature of about 20 to 25 °C and an absolute pressure of about 1 atm.

The slurrying process comprises the steps of:
a) stirring a mixture of bilastine as defined above, or a solvate or polymorph thereof, and the neutral cocrystal forming compound in an appropriate solvent, preferably water, at a temperature between room temperature and 40°C;
b) cooling the mixture to room temperature if temperature of the resulting mixture of the step a) is higher than room temperature, and
c) isolating the obtained compound.

The step a) may be performed by mixing equimolar amounts of each cocrystal former, i.e. of bilastine and the selected coformer, and slurrying the mixture in the appropriate solvent. In a preferred embodiment the solvent is water. Step a) may also be performed by mixing bilastine and an organic carboxylic acid, preferably in an amount needed to obtain a pH of between 3.5 and 5 in water. Step b) is performed only when the obtained slurry of step a) presents a temperature higher than room temperature. The obtained solid suspended in the solvent is isolated in step c). The isolation of the solid may include, for example, one or more of the following operations: filtration, filtration under vacuum, evaporation, decantation, and centrifugation and other suitable techniques as known to a person skilled in the art. The obtained cocrystal of the invention may be purified, e.g. by recrystallization.

The liquid assisted grinding comprises:
a) wet grinding of bilastine, or a solvate or polymorph thereof, and the neutral cocrystal forming compound as defined above in an appropriate solvent, preferably water, and
b) isolating the obtained compound.

The grinding may be performed, for instance, in a ball mill. In a preferred embodiment the solvent is present in catalytic amount. The isolation of the solid may include, for example, one or more of the following operations: filtration, filtration under vacuum, evaporation, decantation, and centrifugation and other suitable techniques as known to a person skilled in the art. The obtained cocrystal of the invention may be further purified, e.g. by recrystallization.

Other processes for obtaining the cocrystals known in the art are also encompassed by this invention, for example, evaporation, crystallization by cooling, and heating-melting.

The process for preparing a cocrystal according to the invention comprises putting in contact at least the first neutral component bilastine and a second neutral cocrystal forming compound. Without wishing to be bound by any particular theory, the process is such that it is believed that the first neutral component bilastine can either exist as a neutral zwitterionic species, wherein the acidic moiety is deprotonated and, simultaneously, the pyridinic nitrogen of the benzimidazole moiety is protonated or, as a neutral species, in which both acidic and benzimidazole moieties are neutral. In either case, the net charge of bilastine in the process of the invention is zero and thus the first component of the cocrystal is indeed neutral. Furthermore, in the process of the present invention, the conditions are such that the cocrystal forming component is neutral and thus believed to lack any charges due to the pKa difference between the two cocrystal components. Therefore, the cocrystal forming component does not form ionic interactions with other molecules. As will be apparent to the skilled person, if the solvent is water, the pH is such that there is no deprotonation of the second cocrystal forming compound while if the solvent is an organic solvent, then there are no species responsible for deprotonating the second cocrystal forming compound.

### Uses

Benzimidazole compounds have been found to be antagonists of histamine H1 receptor and are thus useful in the treatment and/or prevention of diseases known to be susceptible to improvement by antagonism of histamine H1 receptor.

Therefore, an aspect of the invention refers to a pharmaceutical composition comprising a cocrystal of the invention as defined above for use in the treatment and/or prevention of a disorder or disease susceptible to amelioration by antagonism of H1 histamine receptor. Such diseases are, for example, allergic diseases or disorders.

In another aspect, the invention is directed to a pharmaceutical composition comprising a cocrystal of the invention as defined above for use in the treatment and/or prevention of an allergic disease or disorder. Preferably, an allergic disease or disorder selected from rhinitis, conjunctivitis, rhinoconjunctivitis, dermatitis, urticaria and asthma. Preferably, an allergic disease or disorder selected from rhinitis, conjunctivitis, rhinoconjunctivitis and asthma. More preferably, the allergic disease or disorder is selected from the group consisting of rhinitis, conjunctivitis and rhinoconjunctivitis.

The term "treatment" or "to treat" in the context of this specification means administration of a cocrystal or pharmaceutical composition of the invention to ameliorate or eliminate the disease or one or more symptoms associated with said disease. "Treatment" also encompasses ameliorating or eliminating the physiological sequelae of the disease.

The term "ameliorate" in the context of this invention is understood as meaning any improvement on the situation of the patient treated.

The term "prevention" or "to prevent" in the context of this specification means administration of a cocrystal or formulation according to the invention to reduce the risk of acquiring or developing the disease or one or more symptoms associated with said disease.

### Pharmaceutical composition

In one embodiment, the amount of bilastine in the pharmaceutical composition is above 2000 µg/mL, preferably above 2500 µg/mL, preferably above 3000 µg/mL, preferably above 3500 µg/mL, preferably above 4000 µg/mL, preferably above 4500 µg/mL, preferably above 5000 µg/mL, preferably above 5500 µg/mL, preferably above 6000 µg/mL, preferably above 6500 µg/mL, preferably above 7000 µg/mL, preferably above 7500 µg/mL, preferably above 8000 µg/mL, preferably above 8500 µg/mL, preferably above 9000 µg/mL, and more preferably above 10000 µg/mL.

The expression "therapeutically effective amount" refers to the amount of a medicament which when administered supplies an amount of one or more pharmaceutically active agents contained therein to provide a therapeutic benefit in the treatment or management of a disease or disease state.

In a preferred embodiment the pharmaceutical composition of the invention comprises a therapeutically effective amount of bilastine.

The expression "pharmaceutically acceptable" refers to compositions and molecular entities that are physiologically tolerable and do not typically produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The expression "pharmaceutically acceptable excipient" refers to a vehicle, diluent, carrier or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

The excipients and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993.

The following examples are merely illustrative of certain embodiments of the invention and cannot be considered as restricting it in any way.

### EXAMPLES

### Equipment and methods used

### Powder X-Rav Diffraction Analysis

Sample preparation: the non-manipulated samples were mounted on a zero-background silicon holder. Data collection: Diffraction measurements were performed at ambient conditions on a PANalytical X'Pert PRO diffractometer with reflection θ-θ geometry, equipped with Cu K-alpha radiation and a PIXcel detector, operated at 45 kV and 40 mA. The samples were allowed to spin at 0.25 rev/s during the data collection. The measurement angular range was 3.0-40.0° (2θ) with a step size of 0.013°. The scanning speed was 0.3283°/s (10.20 s/step). Programs used: data collection with X'Pert Data Collector v 2.2i and treatment with X'Pert HighScore v 2.2cand X'Pert Data Viewer 1.2d.

### DSC-TGA

Thermogravimetric analyses were recorded in a TA SDT Q600. Samples of 5 mg were weighed (using a microscale AE240, Mettler) into 90 µL open alumina crucibles, and were heated at 10 °C/min between 25 and 300 °C, under a nitrogen flow (50 mL/min). Data collection and evaluation was performed with TA Universal Analysis 2000 v 4.7 software.

### Proton Nuclear Magnetic Resonance (¹H-NMR)

Proton nuclear magnetic resonance analyses were recorded in various deuterated solvents, such as dimethylsulfoxide (DMSO-d₆), methanol (MeOH-d₄) and water (D₂0) in a Varian Mercury 400 spectrometer. Spectra were acquired solving 5-10 mg of sample in 0.6 mL of deuterated solvent.

### Grinding Experiments

The grinding experiments were performed in a Retsch MM400 Ball Mill. The mixtures and the milling balls (stainless steel, diameter: 5 mm) were introduced in 9 position milling jars (stainless steel, cell volume: 1.5 mL), the solvent was added to each mixture with a 10 µL microsyringe and the jars were immediately introduced in the clamping device. The samples were then subjected to a 30 min grinding cycle (frequency: 30 s⁻¹).

### Fourier Transformed Infrared Spectroscopy Analysis

The FTIR spectra were recorded using a Bruker Alpha spectrometer, equipped with a Bruker Diamond single reflection ATR system, a mid-infrared source as the excitation source and a DTGS detector. The spectra were acquired in 32 scans at a resolution of 4 cm⁻¹ in the range of 4000-400 cm⁻¹.

### Solubility

Solubility of cocrystals was determined by stirring the product in water at room temperature (400 mg of product in 24 ml of water, 60 vol.). The product was previously milled in order to reduce the crystal size effect. A sample is collected and filtered using a sintered funnel (n° 3) periodically (30, 60, 180 min and after overnight). The filtered solid was immediately analyzed by XRPD, while the mother liquors are filtered through a 0.22 um filter and the concentration of Bilastine is determined by HPLC area (in some experiments where a high solubility was detected the mother liquor was diluted by a factor 10). This concentration is compared with the Bilastine concentration obtained in the same conditions in order to determine a relative solubility.

HPLC analyses were performed by duplicate on an Agilent 1100 series apparatus with a XBridge C-18 column at room temperature. 25 µL sample were injected. The mobile phase (isocratic) was set as following: 52%(Methanol : aqueous octylamine 0.01M pH = 7, 65:35) - 42% (Acetonitrile : aqueous octylamine 0.01M pH = 7, 60:40)

### Stability

Stability of the cocrystal was determined by storing a sample of this product under atmospheric and forced conditions according to ICH guideline (40°C, 70% ± 5% HR). The solids were analyzed by XRPD every day during the first week and once a week afterwards, to detect possible crystalline transformations.

### Example 1. Bilastine/ glutaric acid cocrystals

### Example 1.1. Crystalline form GL(I)_bilastine/glutaric acid 2:1 cocrystal

The crystalline form, named as GL(I), was obtained by wet grinding of a bilastine: glutaric acid 1:2 mixture in water.

This crystalline form was also obtained by slurrying bilastine (120 mg) and glutaric acid at a stoichiometry ratio of 1:1 or 1:2 in water (15 mL). After stirring 15 hours, the obtained solid suspended in the water was isolated by filtration, washed with water and dried under vacuum.

This crystalline form was also obtained by seeding with GL(I) a suspension of BLN(I) (1000 mg, 2.157 mmol) and glutaric acid (570.0 mg, 4.314 mmol) in water (10 mL), at rt. After stirring 18 hours at rt, the obtained solid suspended in water was isolated by filtration, washed with water (2 x 1 mL) and dried under vacuum over 18 hours, to yield 974 mg of GL(I) as a white solid (yield 81%).

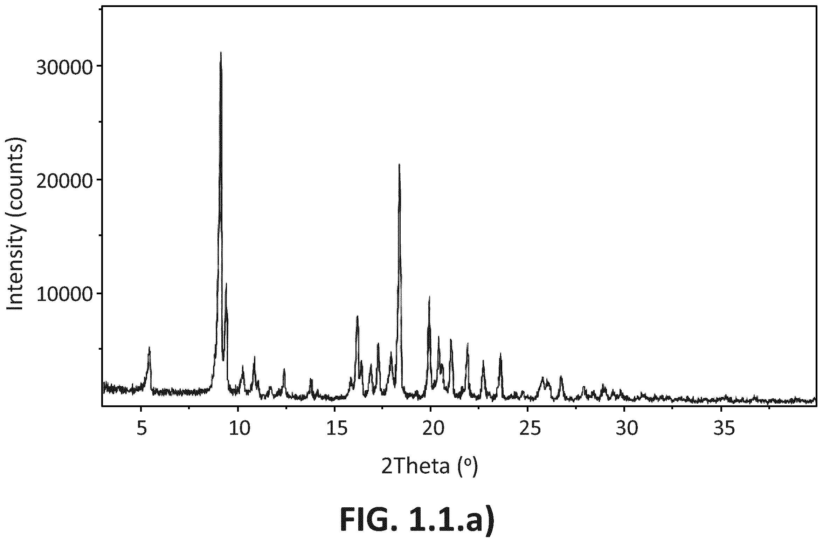

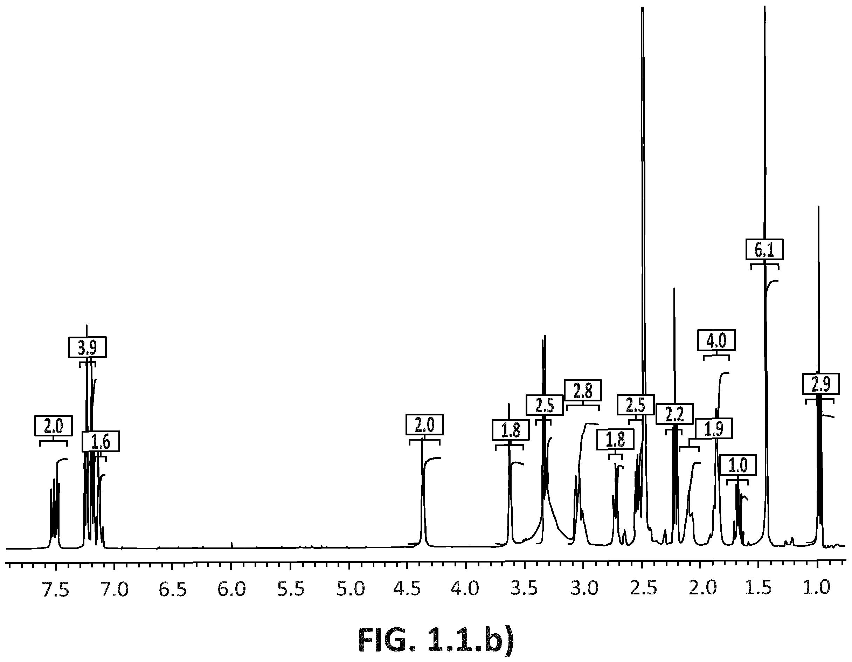

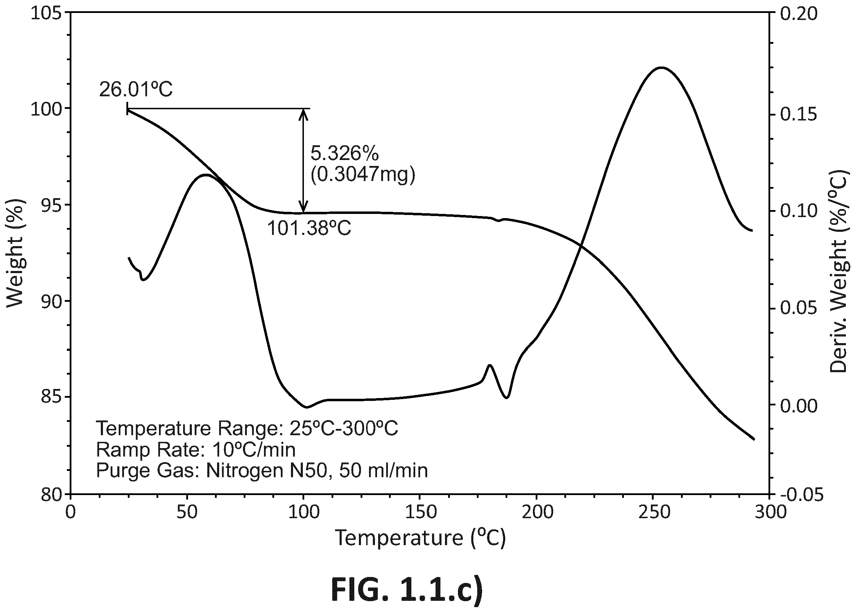

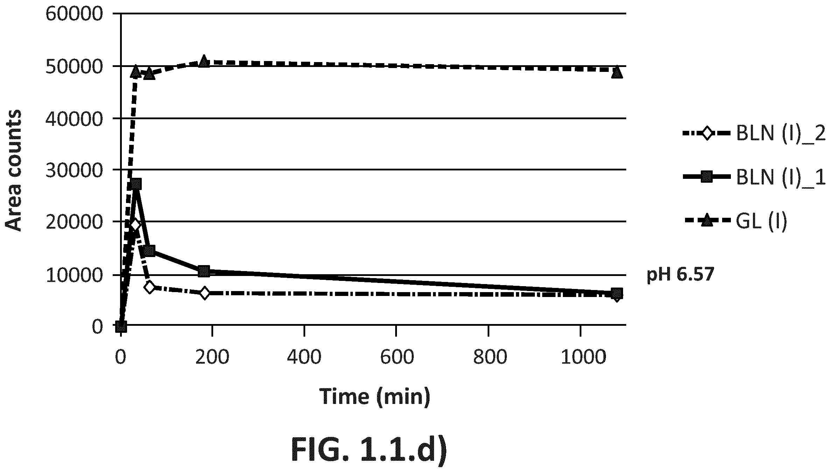

The resulting cocrystal was characterised by XPRD (see Fig. 1.1, a)), ¹H-NMR (see Fig. 1.1, b)), and TGA (see Fig. 1.1, c)). XPDR analysis confirmed that this form is stable under forced conditions of humidity and temperature (70 ± 5% RH, 40 °C) for at least 4 weeks. A 2:1 ratio of bilastine:glutaric acid was confirmed by ¹H-NMR.

**Table 1.1. XRPD peak list of GL(I):**

| Pos. [°2Th.] | Rel. Int. [%] |
|---|---|
| 5.44 | 12 |
| 9.13 | 100 |
| 9.41 | 32 |
| 10.28 | 8 |
| 10.89 | 12 |
| 11.72 | 2 |
| 12.42 | 8 |
| 13.82 | 5 |
| 15.85 | 4 |
| 16.19 | 22 |
| 16.42 | 9 |
| 16.91 | 9 |
| 17.29 | 15 |
| 17.96 | 11 |
| 18.41 | 67 |
| 19.93 | 27 |
| 20.40 | 17 |
| 20.59 | 9 |
| 21.06 | 17 |
| 21.88 | 15 |
| 22.73 | 10 |
| 23.60 | 12 |
| 24.77 | 2 |
| 25.79 | 6 |
| 26.14 | 3 |
| 26.70 | 7 |
| 27.90 | 4 |
| 28.39 | 1 |
| 28.99 | 3 |
| 29.84 | 2 |
| 31.02 | 2 |
| 35.27 | 1 |

This cocrystal improves greatly the solubility of bilastine as shown in Figure 1.1.d)

### Example 1.2. Crystalline form GL(IV)_bilastine:glutaric acid 2:1 cocrystal

This crystalline form was obtained by slurrying bilastine and glutaric acid at a stoichiometry ratio of 1:1, 1:2 or 2:1 in ethanol. After stirring for 15 hours, the obtained solid suspended in ethanol was isolated by filtration, washed with ethanol and dried under vacuum.

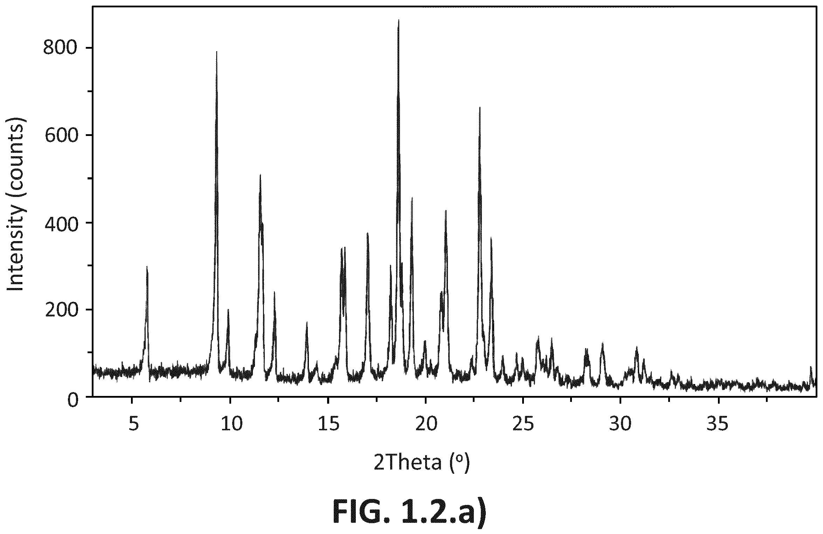

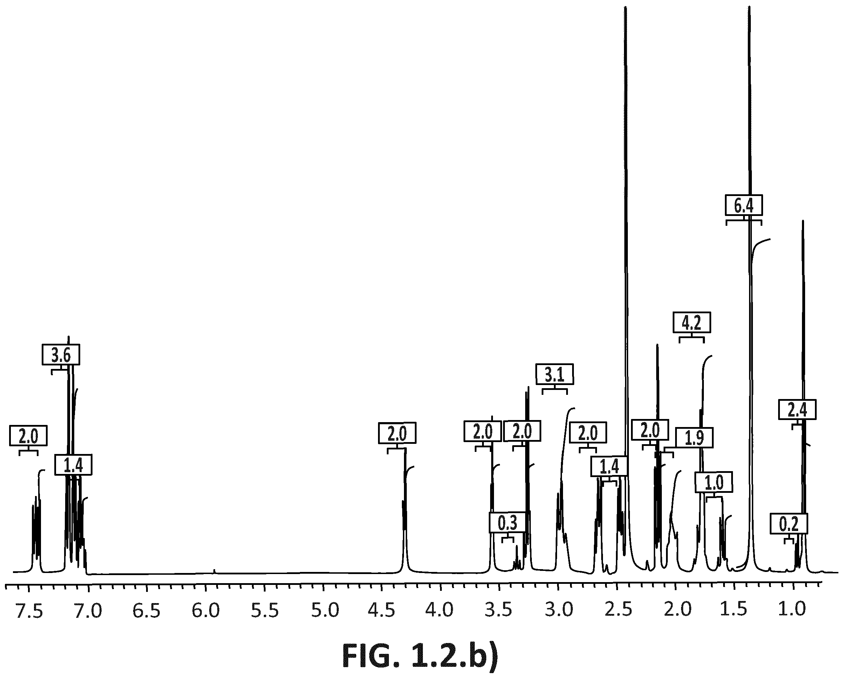

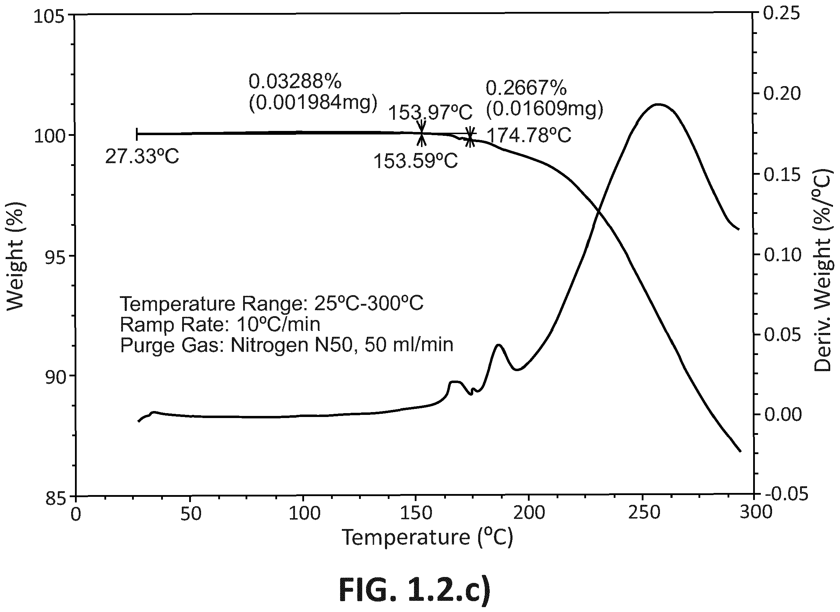

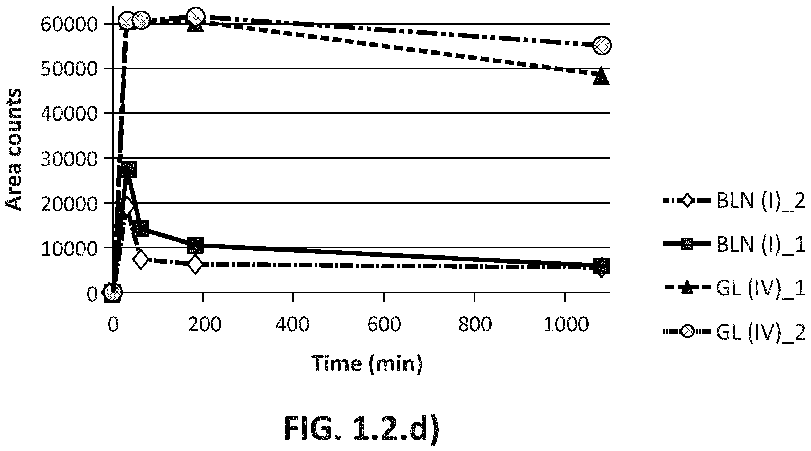

The resulting cocrystal according to this example was characterised by XPRD (see Fig. 1.2, a)) and ¹H-NMR (see Fig. 1.2, b)).

This crystalline form was also obtained by seeding with GL(IV) a suspension of BLN(I) (1000 mg, 2.157 mmol) and glutaric acid (285.0 mg, 2.157 mmol) in EtOH (10 mL), at rt. After stirring 18 hours at rt, the obtained solid suspended in water was isolated by filtration, washed with water (2 x 1 mL) and dried under vacuum over 18 hours, to yield 1.079 mg of GL(IV) as a white solid (yield 94%).

The resulting cocrystal was characterised by XPRD (see Fig. 1.2, a)), ¹H-NMR (see Fig. 1.2, b)) and TGA (see Fig. 1.2, c)). XPDR analysis confirmed that this form is stable under forced conditions of humidity and temperature (70 ± 5% RH, 40 °C) for at least 4 weeks. A 2:1 ratio of bilastine:glutaric acid was confirmed by ¹H-NMR.

**Table 1.2. XRPD peak list of GL (IV):**

| Pos. [°2Th.] | Rel. Int. [%] |
|---|---|
| 3.18 | 2 |
| 5.85 | 31 |
| 9.39 | 82 |
| 9.97 | 17 |
| 11.61 | 60 |
| 11.73 | 45 |
| 12.33 | 25 |
| 13.98 | 17 |
| 14.50 | 4 |
| 15.45 | 9 |
| 15.73 | 37 |
| 15.93 | 35 |
| 17.10 | 41 |
| 18.29 | 27 |
| 18.67 | 100 |
| 18.85 | 33 |
| 19.36 | 52 |
| 20.03 | 9 |
| 20.86 | 23 |
| 21.08 | 45 |
| 22.40 | 4 |
| 22.82 | 81 |
| 23.42 | 42 |
| 24.00 | 7 |
| 24.70 | 7 |
| 25.01 | 7 |
| 25.77 | 11 |
| 26.22 | 6 |
| 26.51 | 10 |
| 26.78 | 4 |
| 28.21 | 8 |
| 28.37 | 7 |
| 29.08 | 12 |
| 30.80 | 9 |
| 31.21 | 7 |
| 32.60 | 3 |
| 32.95 | 3 |
| 35.93 | 2 |
| 37.84 | 1 |
| 39.75 | 6 |

This cocrystal improves greatly the solubility of bilastine as shown in Figure 1.2.d).

### Example 1.3. Crystalline form GL(V)_bilastine/ glutaric acid 1:1 cocrystal

This crystalline form was obtained by slurrying bilastine and glutaric acid at a stoichiometry ratio of 1:2 in MIK, AcOⁱBu or TBME.

This crystalline form was also obtained by slurrying bilastine and glutaric acid at a stoichiometry ratio of 1:1 or 1:2 in TBME or ACN. After stirring 18 hours, the obtained solid suspended in the solvent was isolated by filtration, washed with the same solvent and dried under vacuum.

This crystalline form was also obtained by seeding with GL(V) a suspension of BLN(I) (1000 mg, 2.157 mmol) and glutaric acid (570.0 mg, 4.314 mmol) in ACN (10 mL), at rt. After stirring 18 hours, the obtained solid suspended in ACN was isolated by filtration, washed with ACN (2 x 1 mL) and dried under vacuum over 18 hours, to yield 1.157 mg of GL(V) as a white solid.

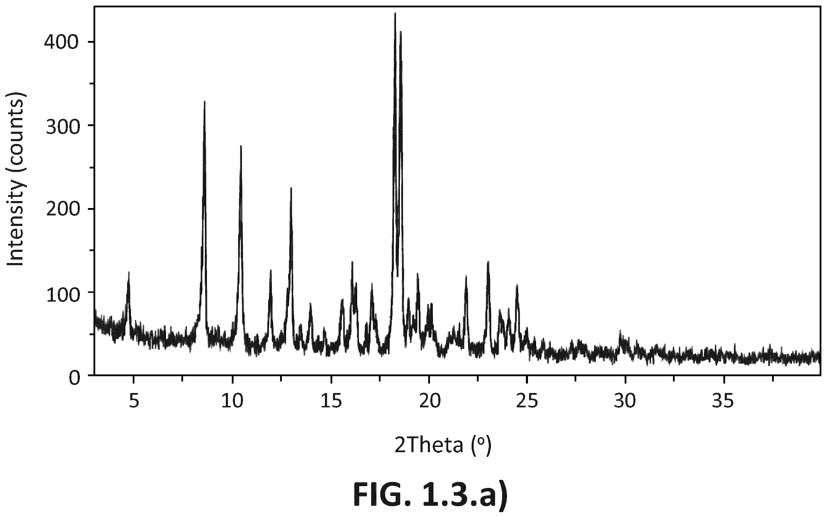

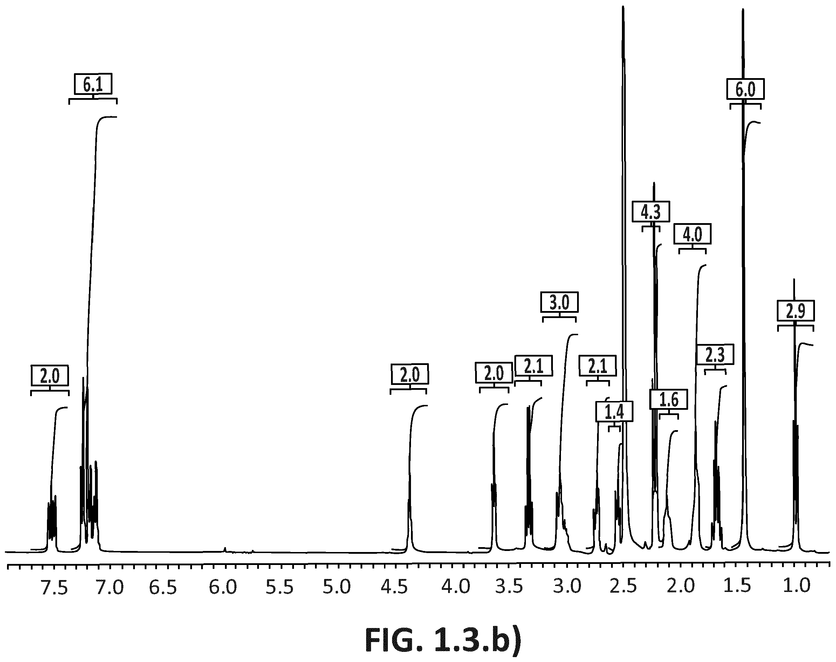

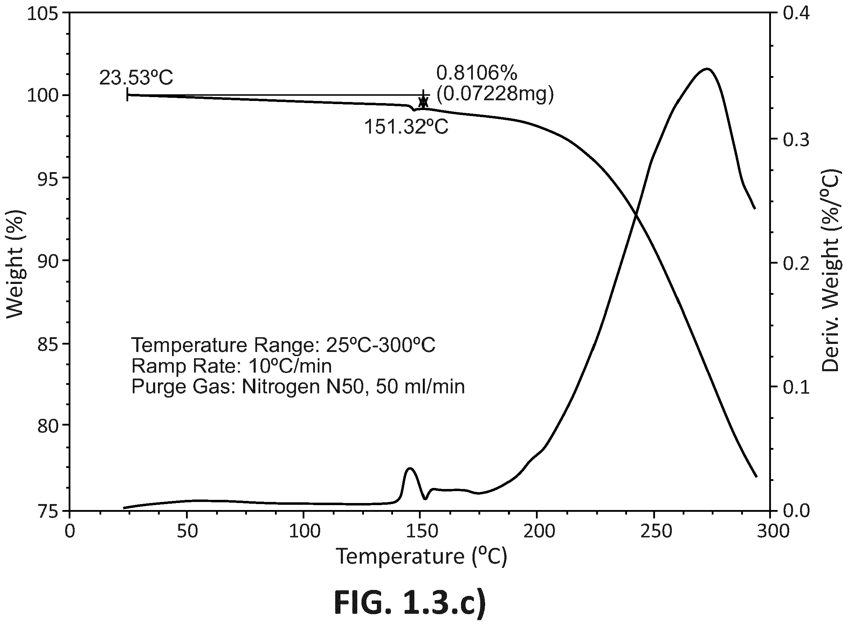

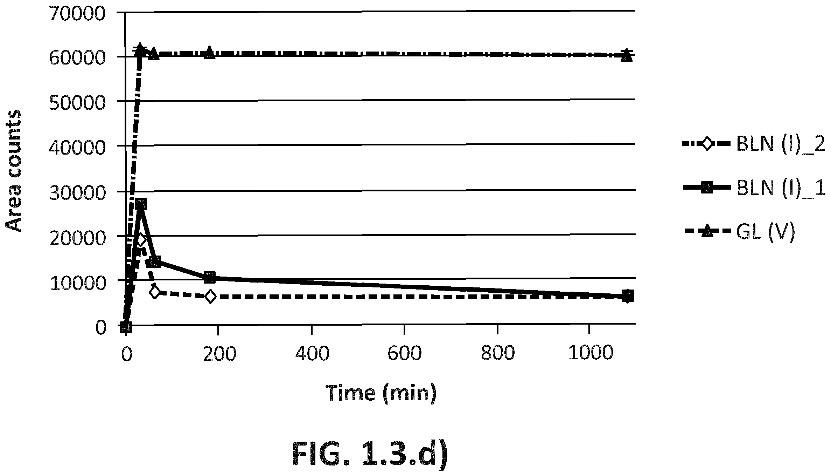

The resulting cocrystal was characterised by XPRD (see Fig. 1.3, a)), ¹H-NMR (see Fig. 1.3, b)) and TGA (see Fig. 1.3 c)). XPDR analysis confirmed that this form is stable under forced conditions of humidity and temperature (70 ± 5% RH, 40 °C) for at least 4 weeks. A 1:1 ratio of bilastine:glutaric acid was confirmed by ¹H-NMR.

**Table 1.3. XRPD peak list of GL (V):**

| Pos. [°2Th.] | Rel. Int. [%] |
|---|---|
| 4.75 | 18 |
| 8.63 | 76 |
| 10.47 | 64 |
| 12.00 | 22 |
| 13.02 | 46 |
| 13.50 | 6 |
| 14.03 | 12 |
| 14.73 | 5 |
| 15.64 | 15 |
| 16.12 | 24 |
| 16.33 | 19 |
| 16.88 | 7 |
| 17.14 | 17 |
| 18.34 | 96 |
| 18.62 | 100 |
| 19.02 | 14 |
| 19.50 | 23 |
| 19.96 | 9 |
| 20.19 | 12 |
| 21.19 | 4 |
| 21.94 | 22 |
| 23.05 | 27 |
| 23.66 | 12 |
| 24.12 | 11 |
| 24.55 | 21 |
| 25.03 | 7 |
| 25.89 | 4 |
| 27.66 | 2 |
| 29.79 | 7 |
| 30.60 | 3 |
| 31.58 | 3 |

This cocrystal improves greatly the solubility of bilastine as shown in Figure 1.3.d).

### Example 2. Bilastine/ adipic acid cocrystals

### Example 2.1. Crystalline form AD(I)_Bilastine/ adipic acid 1:1

This crystalline form was obtained by slurrying bilastine and adipic acid at a stoichiometry ratio of 1:2 in MeOH. After stirring 15 hours, the obtained solid suspended in MeOH was isolated by filtration, washed with MeOH and dried under vacuum.

This crystalline form was also obtained by slurrying bilastine and adipic acid at a stoichiometry ratio of 1:1 in AcOEt or ACN.

The crystalline form was also obtained by wet grinding at a stoichiometry ratio of 1:2 in MeOH, AcOEt or ACN.

This crystalline form was also obtained by seeding with AD(I) a suspension of BLN(I) (1000 mg, 2.157 mmol) and adipic acid (630.4 mg, 4.314 mmol) in MeOH (10 mL), at rt. After stirring 18 hours, the obtained solid suspended in MeOH is isolated by filtration, washed with MeOH (2 x 1 mL) and dried under vacuum over 18 hours, to yield 1.157 mg of AD(I) as a white solid.

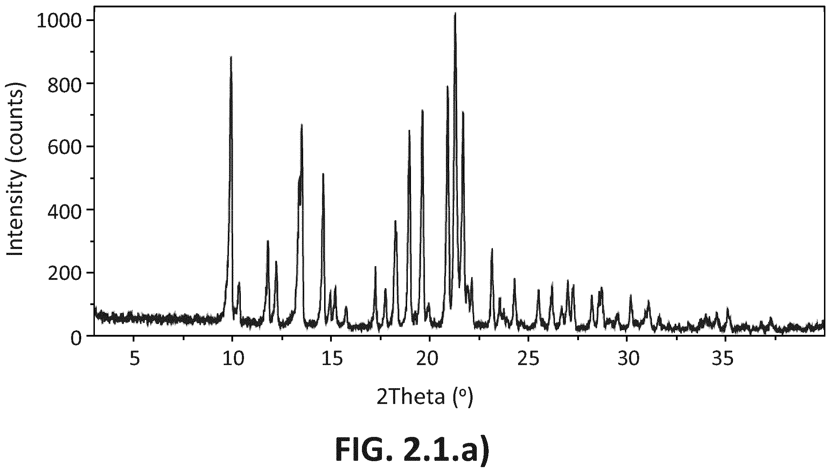

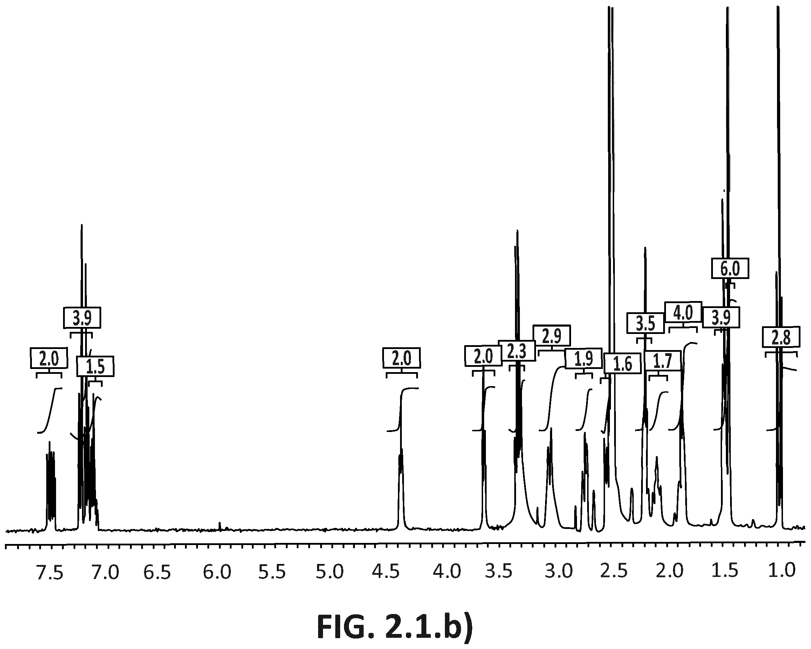

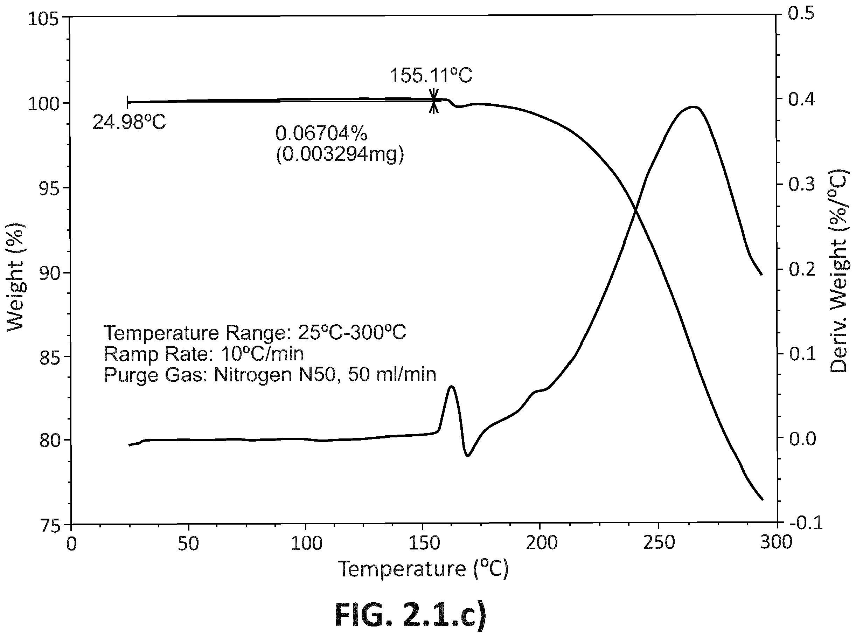

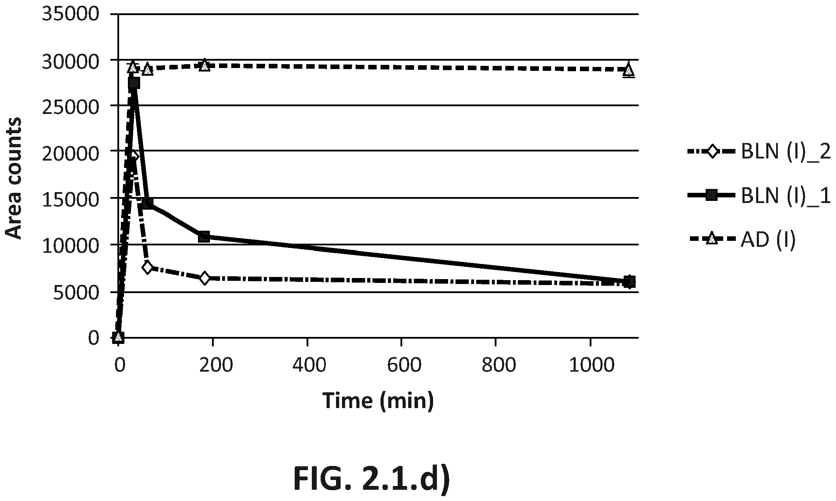

The resulting cocrystal was characterised by XPRD (see Fig. 2.1, a)), ¹H-NMR (see Fig. 2.1, b)) and TGA (see Fig. 2.1 c)). XPDR analysis confirmed that this form is stable under forced conditions of humidity and temperature (70 ± 5% RH, 40 °C) for at least 1 week. A 1:1 ratio of bilastine:adipic acid was confirmed by ¹H-NMR.

**Table 2.1. XRPD peak list of AD(I):**

| Pos. [°2Th.] | Rel. Int. [%] |
|---|---|
| 9.93 | 81 |
| 10.34 | 12 |
| 11.79 | 26 |
| 12.21 | 19 |
| 13.34 | 45 |
| 13.52 | 57 |
| 14.59 | 46 |
| 14.94 | 9 |
| 15.20 | 11 |
| 15.76 | 5 |
| 17.24 | 18 |
| 17.78 | 10 |
| 18.24 | 30 |
| 18.33 | 23 |
| 18.97 | 59 |
| 19.60 | 67 |
| 19.91 | 6 |
| 20.89 | 76 |
| 21.27 | 100 |
| 21.68 | 68 |
| 21.90 | 12 |
| 22.10 | 14 |
| 23.13 | 24 |
| 23.52 | 9 |
| 24.27 | 15 |
| 25.49 | 12 |
| 26.17 | 13 |
| 26.64 | 6 |
| 26.97 | 15 |
| 27.19 | 12 |
| 28.17 | 8 |
| 28.52 | 8 |
| 28.69 | 12 |
| 29.06 | 2 |
| 29.50 | 4 |
| 30.15 | 9 |
| 31.06 | 8 |
| 31.55 | 3 |
| 33.09 | 2 |
| 33.93 | 3 |
| 34.51 | 4 |
| 35.06 | 6 |
| 36.77 | 2 |
| 37.23 | 3 |
| 38.20 | 1 |
| 39.18 | 1 |

This cocrystal improves greatly the solubility of bilastine as shown in Figure 2.1.d).

### Example 2.2. Crystalline form AD(III)_Bilastine:adipic acid 2:1

This crystalline form was obtained by slurrying bilastine (120 mg) and adipic acid at a stoichiometry ratio of 1:1 or 1:2 in water (15 mL). The suspension was heated to reflux (without dissolving the suspended solid) and cooled to rt. After stirring 15 hours at rt, the obtained solid suspended in the water was isolated by filtration, washed with water and dried under vacuum.

This crystalline form was also obtained by seeding with AD(III) a suspension of BLN(I) (1000 mg, 2.157 mmol) and adipic acid (473.0 mg, 3.235 mmol) in water (10 mL), that has been cooled to rt after being heated to reflux. After stirring 18 hours at rt, an analysis showed free adipic acid. 200 mg (0.431 mmol) of BLN(I) were further added. The mixture is heated again to reflux and cooled to rt. After stirring other 18 hours at rt, the obtained solid suspended in water is isolated by filtration, washed with water (2 x 1 mL) and dried under vacuum over 18 hours, to yield 1.330 mg of AD (III) as a white solid (yield 91%).

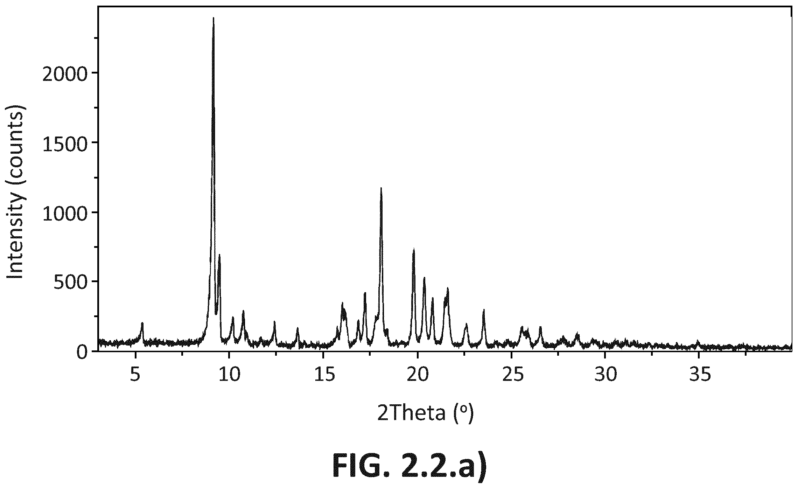

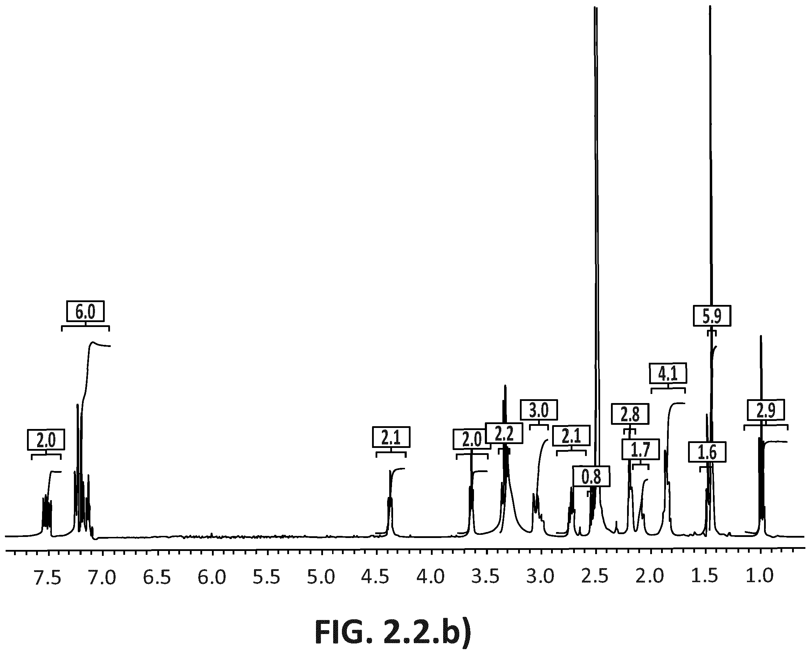

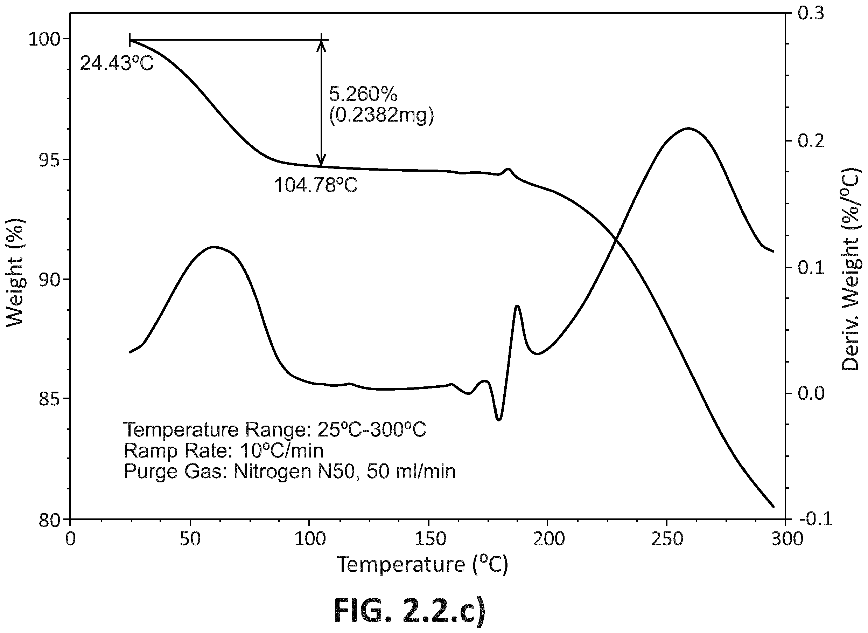

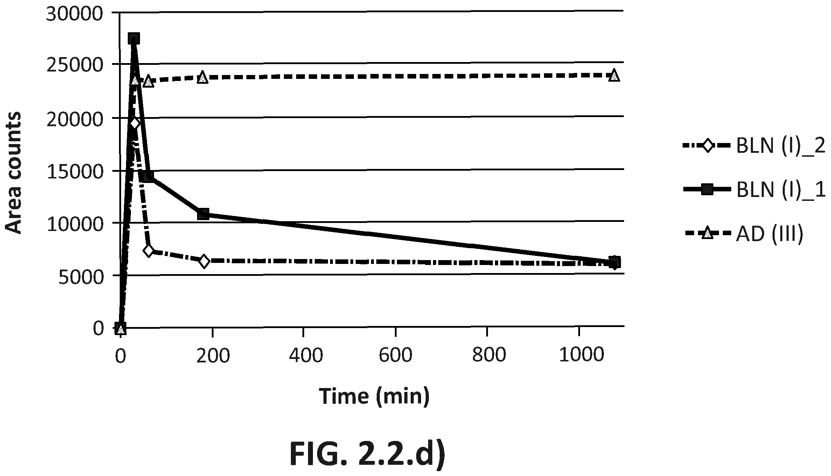

The resulting cocrystal was characterised by XPRD (see Fig. 2.2, a)), ¹H-NMR (see Fig. 2.2, b)) and TGA (see Fig. 2.2, c)). XPDR analysis confirmed that this form is stable under forced conditions of humidity and temperature (70 ± 5% RH, 40 °C) for at least 1 week. A 2:1 ratio of bilastine:adipic acid was confirmed by ¹H-NMR.

**Table 2.2. XRPD peak list of AD(III):**

| Pos. [°2Th.] | Rel. Int. [%] |
|---|---|
| 5.38 | 7 |
| 9.16 | 100 |
| 9.48 | 26 |
| 10.19 | 7 |
| 10.75 | 10 |
| 11.66 | 2 |
| 12.40 | 8 |
| 13.63 | 6 |
| 14.00 | 1 |
| 15.78 | 4 |
| 16.02 | 12 |
| 16.19 | 10 |
| 16.89 | 7 |
| 17.25 | 16 |
| 17.79 | 8 |
| 18.10 | 51 |
| 18.43 | 5 |
| 19.79 | 30 |
| 20.38 | 22 |
| 20.83 | 14 |
| 21.45 | 13 |
| 21.63 | 18 |
| 22.63 | 7 |
| 23.53 | 11 |
| 24.82 | 1 |
| 25.57 | 5 |
| 25.92 | 4 |
| 26.57 | 6 |
| 27.84 | 2 |
| 28.48 | 4 |
| 29.31 | 2 |
| 31.11 | 1 |
| 34.97 | 1 |

This cocrystal improves greatly the solubility of bilastine as shown in Figure 2.2.d).

### Example 3. Bilastine/ sorbic acid cocrystals

### Example 3.1. Crystalline form SO(I)_bilastine/ sorbic acid 2:1 cocrystal

The crystalline form was obtained by wet grinding of a 1:1 stoichiometric ratio of bilastine:sorbic acid in water.

This crystalline form was also obtained by slurrying bilastine (120 mg) and sorbic acid at a stoichiometry ratio of 1:1, 2:1 or 1:2 in water (15 mL).

This crystalline form was also obtained by adding water (8 mL) to a mixture of bilastine (1g, 2.16 mmol) and sorbic acid (1 eq, 2.16 mmol). The resulting suspension was seeded with SO(I) and stirred for 20 hours at rt. The obtained solid was isolated by filtration, washed with water (2 x 1 mL) and TBME (4 x 2 mL) and dried under vacuum, to yield 1.03 g of SO(I) (yield 92%).

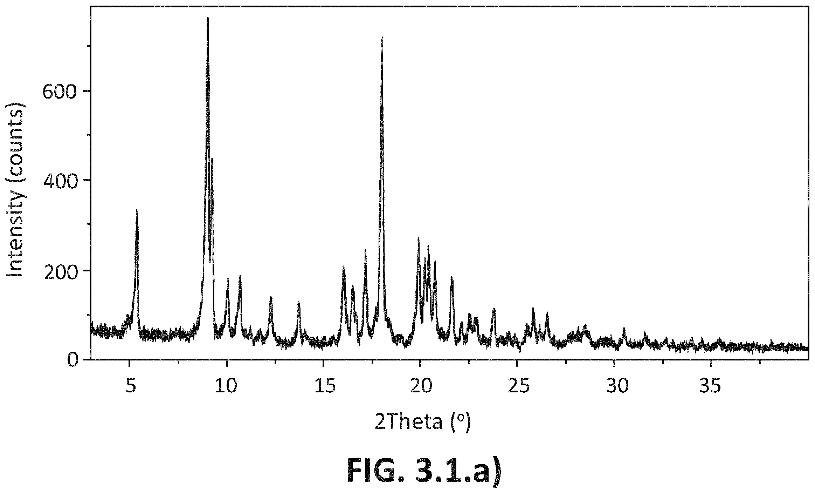

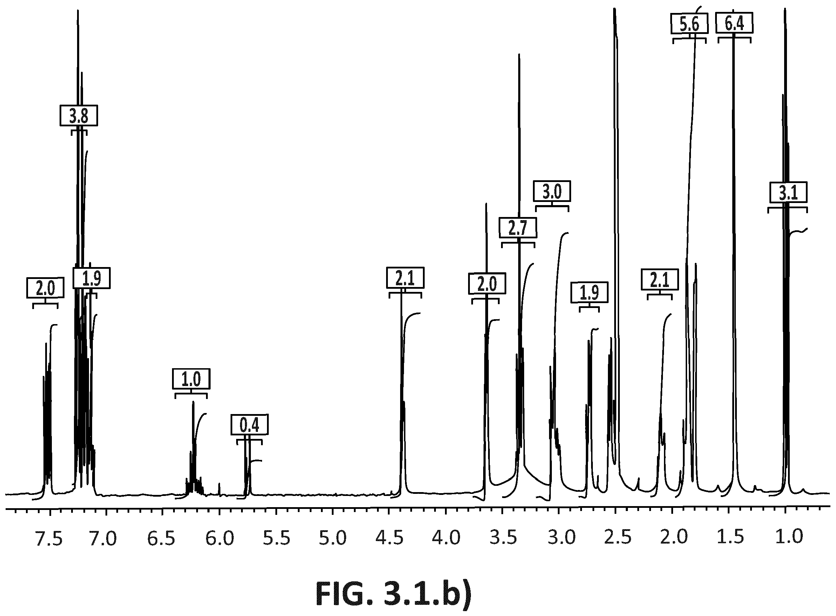

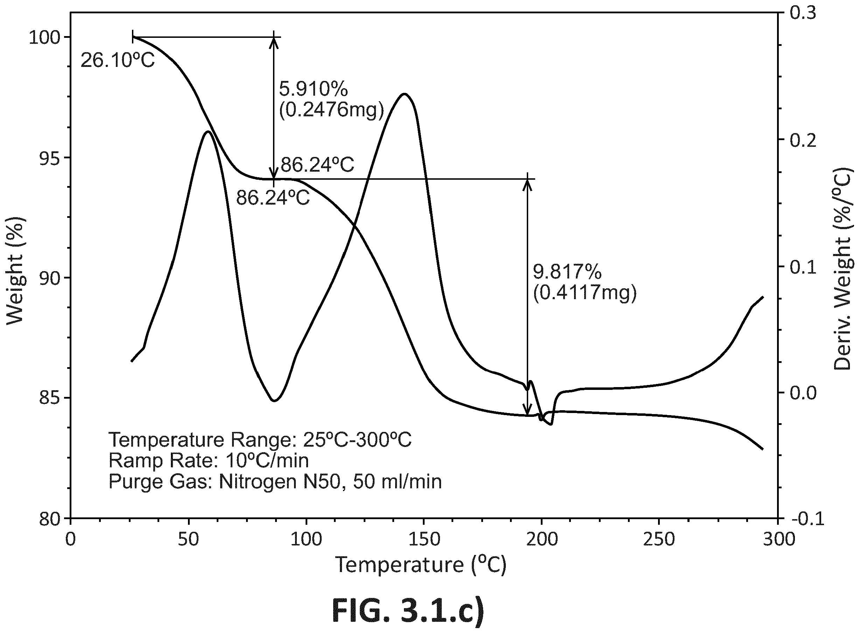

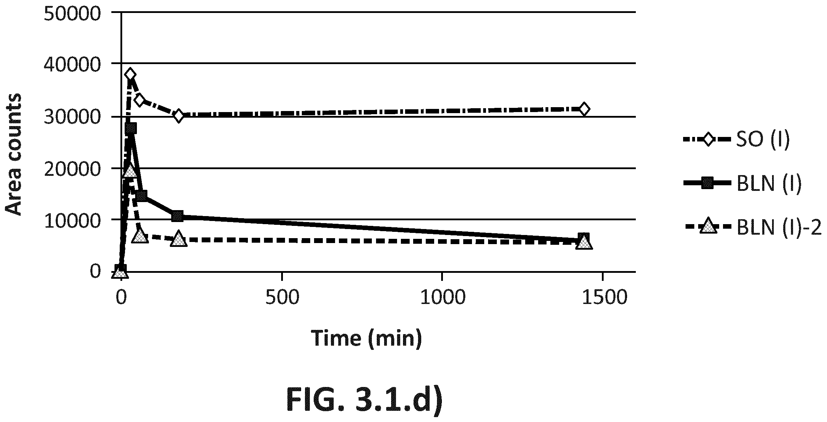

The resulting cocrystal was characterised by XPRD (see Fig. 3.1, a)), ¹H-NMR (see Fig. 3.1, b)) and TGA (see Fig. 3.1, c)). XPDR analysis confirmed that this form was stable forced conditions of humidity and temperature (75 ± 5% RH, 40 °C) for at least 1 week. A 2:1 ratio of bilastine:sorbic acid was confirmed by ¹H-NMR.

**Table 3.1. XRPD peak list of SO(I):**

| Pos. [°2Th.] | Rel. Int. [%] |
|---|---|
| 5.49 | 33 |
| 9.11 | 96 |
| 9.34 | 49 |
| 10.13 | 18 |
| 10.72 | 18 |
| 11.83 | 3 |
| 12.37 | 8 |
| 13.79 | 14 |
| 16.06 | 24 |
| 16.54 | 16 |
| 16.73 | 8 |
| 17.20 | 22 |
| 17.69 | 9 |
| 18.06 | 100 |
| 18.33 | 6 |
| 19.95 | 31 |
| 20.27 | 24 |
| 20.49 | 28 |
| 20.76 | 25 |
| 21.64 | 23 |
| 22.13 | 5 |
| 22.58 | 7 |
| 22.92 | 7 |
| 23.77 | 12 |
| 24.60 | 1 |
| 25.47 | 5 |
| 25.89 | 7 |
| 26.16 | 4 |
| 26.51 | 9 |
| 27.67 | 2 |
| 28.16 | 4 |
| 28.49 | 4 |
| 29.23 | 1 |
| 30.47 | 3 |
| 31.56 | 3 |
| 32.52 | 1 |
| 34.57 | 1 |
| 35.31 | 1 |
| 38.14 | 1 |

This cocrystal improves greatly the solubility of bilastine as shown in Figure 3.1.d).

### Example 4. Bilastine/ succinic acid cocrystals

### Example 4.1. Crystalline form SU(VI)_bilastine/ succinic acid 1:1 cocrystal

The crystalline form was obtained by slurrying bilastine and succinic acid at a stoichiometry ratio of 1:2 or 2:3 in ACN.

This crystalline form was also obtained by slurrying bilastine (120 mg) and succinic acid at a stoichiometry ratio of 1:1, 2:1 or 1:2 in water (15 mL).

This crystalline form was also obtained by adding ACN (8 mL) to a mixture of bilastine (1g, 2.16 mmol) and succinic acid (0.38 g, 1.5 eq, 3.23 mmol). The resulting suspension was seeded with SU(VI) and stirred for 20 hours at rt. The obtained solid was isolated by filtration, washed with acetonitrile (2 x 1 mL) and cold acetone (3 x 3 mL) and dried under vacuum, to yield 1.15 g of SU(VI) (yield 92%).

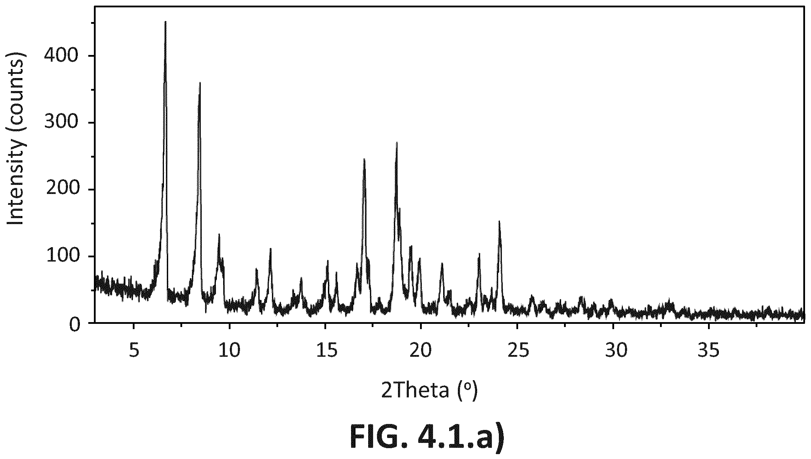

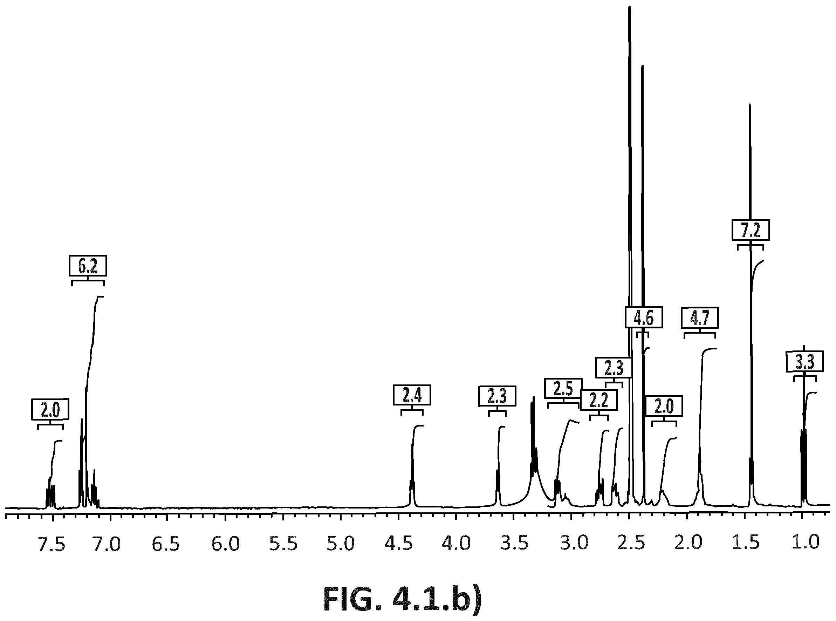

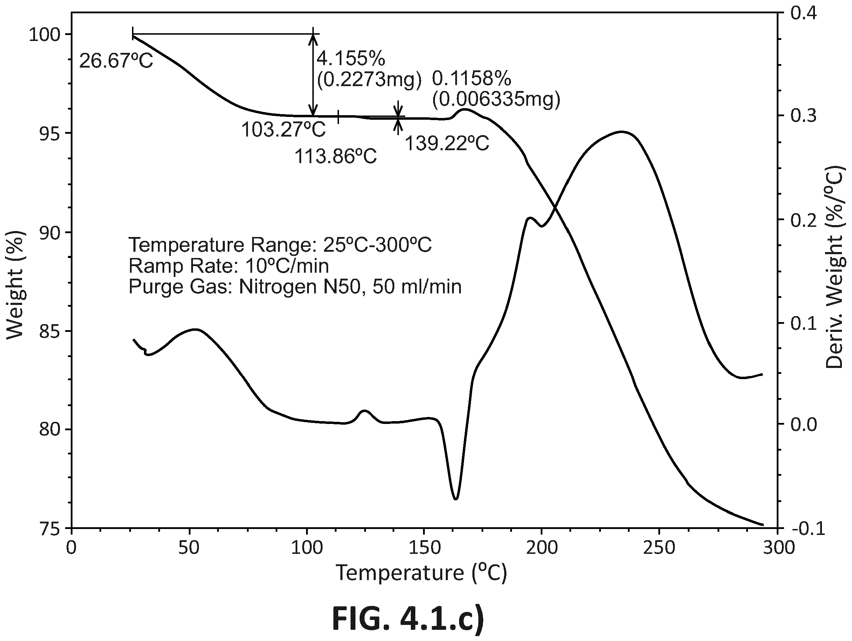

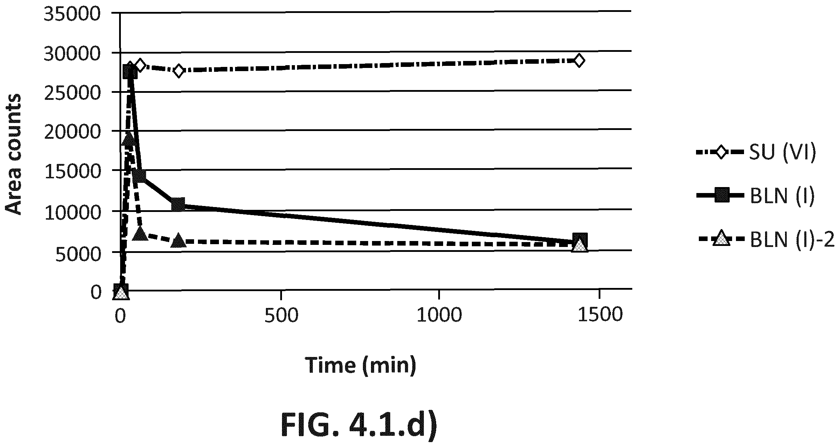

The resulting cocrystal was characterised by XPRD (see Fig. 4.1, a)) and ¹H-NMR (see Fig. 4.1, b)) and TGA (Fig. 4.1, c)). XPDR analysis confirmed that this form was stable forced conditions of humidity and temperature (75 ± 5% RH, 40 °C) for at least 1 week. A 1:1 ratio of bilastine:succinic acid was confirmed by ¹H-NMR.

**Table 4.1. XRPD peak list of SU(VI):**

| Pos. [°2Th.] | Rel. Int. [%] |
|---|---|
| 6.76 | 100 |
| 8.51 | 83 |
| 9.50 | 24 |
| 9.72 | 14 |
| 11.48 | 13 |
| 12.18 | 25 |
| 13.77 | 13 |
| 15.16 | 19 |
| 15.66 | 9 |
| 16.69 | 17 |
| 17.07 | 59 |
| 17.31 | 16 |
| 17.81 | 3 |
| 18.74 | 62 |
| 18.91 | 34 |
| 19.48 | 24 |
| 19.93 | 19 |
| 21.11 | 17 |
| 21.51 | 7 |
| 22.49 | 3 |
| 23.02 | 20 |
| 23.39 | 3 |
| 23.66 | 7 |
| 24.10 | 32 |
| 25.79 | 6 |
| 26.35 | 4 |
| 27.09 | 3 |
| 27.51 | 2 |
| 28.31 | 6 |
| 28.99 | 4 |
| 29.88 | 4 |
| 30.83 | 2 |
| 32.93 | 3 |
| 36.30 | 2 |

This cocrystal improves greatly the solubility of bilastine as shown in Figure 4.1.d).

### Example 4.2. Crystalline form SU(VII)_bilastine/ succinic acid 2:1 cocrystal

The crystalline form was obtained by slurrying bilastine and succinic acid at a stoichiometry ratio of 1:1, 1:2 or 2:1 in EtOH. After stirring for 15 h, the suspension was filtered and the solid washed with EtOH and dried under vacuum.

This crystalline form was also obtained by adding EtOH (8 mL) to a mixture of bilastine (1g, 2.16 mmol) and succinic acid (0.13 g, 0.5 eq, 1.10 mmol). The resulting suspension was seeded with SU(VII) and stirred for 20 hours at rt. The obtained solid was isolated by filtration, washed with ethanol (2 x 1 mL) and dried under vacuum, to yield 1.06 g of SU(VII) (yield 94%).

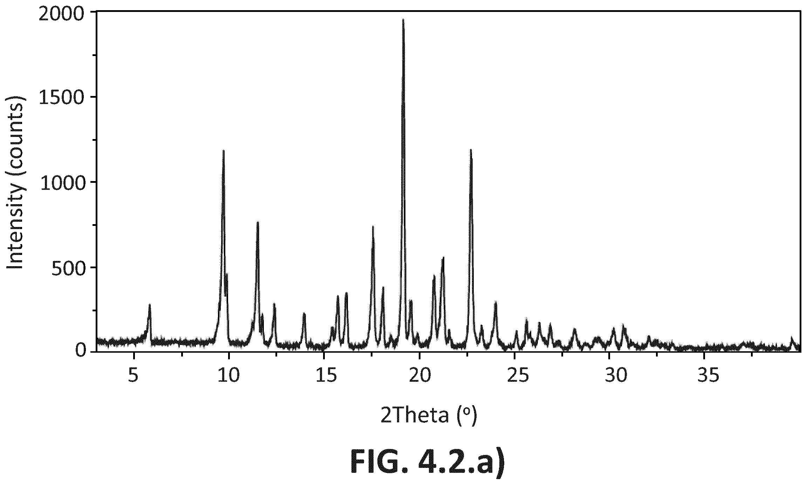

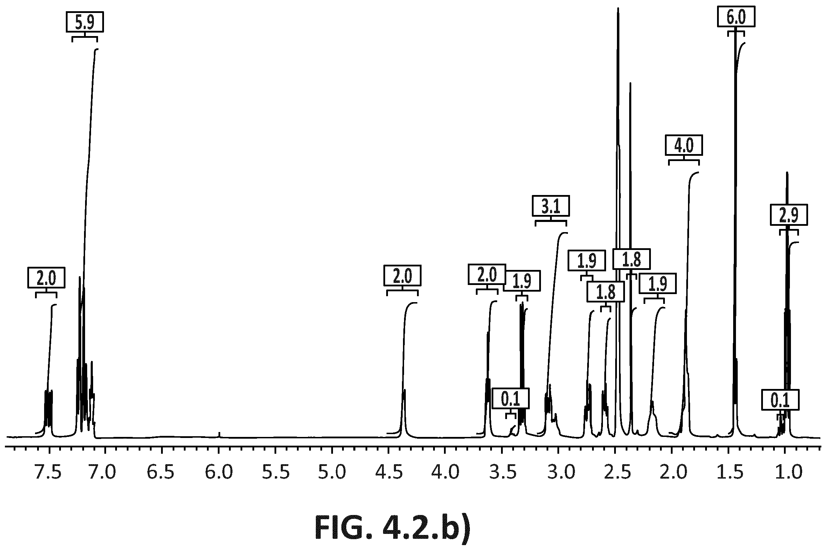

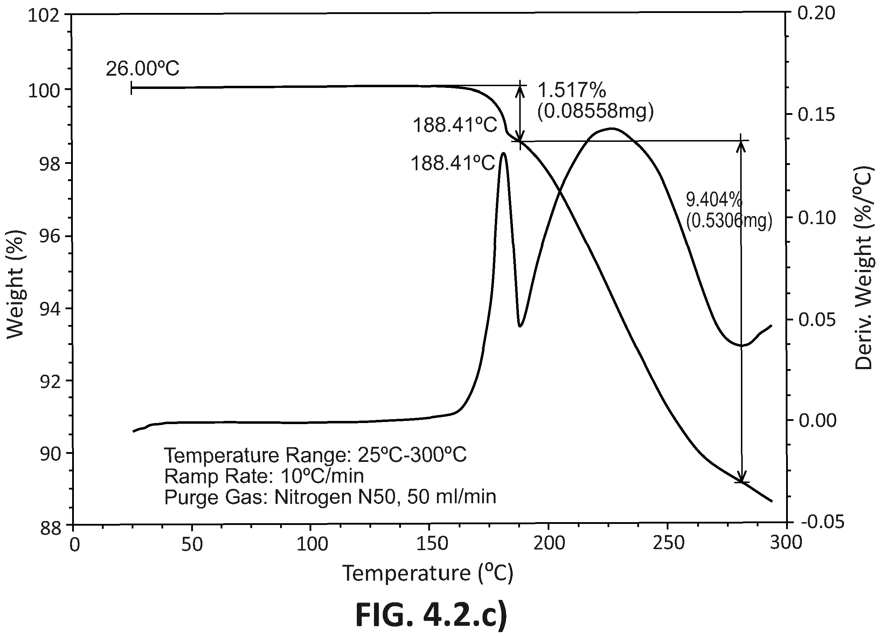

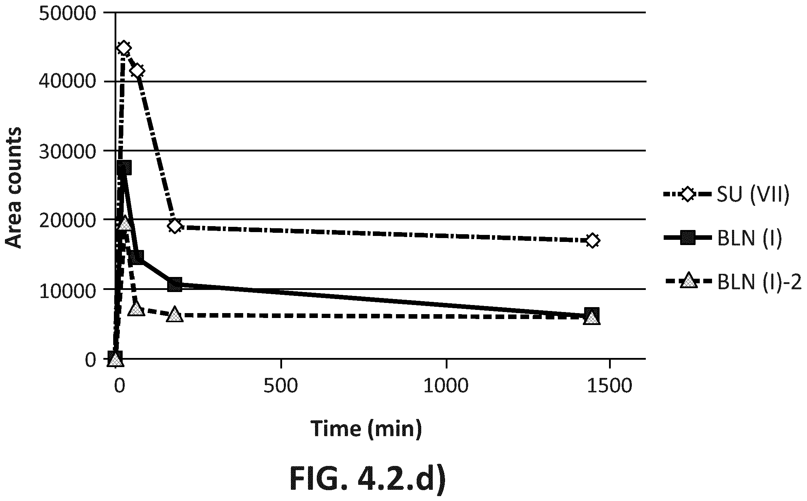

The resulting cocrystal was characterised by XPRD (see Fig. 4.2, a)), ¹H-NMR (see Fig. 4.2, b)) and TGA (Fig. 4.2, c)). XPDR analysis confirmed that this form was stable forced conditions of humidity and temperature (75 ± 5% RH, 40 °C) for at least 1 week. A 2:1 ratio of bilastine:succinic acid was confirmed by ¹H-NMR.

**Table 4.2. XRPD peak list of SU(VII):**

| Pos. [°2Th.] | Rel. Int. [%] |
|---|---|
| 5.88 | 11 |
| 9.74 | 53 |
| 9.91 | 20 |
| 11.54 | 36 |
| 11.78 | 8 |
| 12.42 | 12 |
| 13.98 | 10 |
| 15.42 | 5 |
| 15.74 | 14 |
| 16.20 | 16 |
| 17.58 | 34 |
| 18.10 | 17 |
| 18.51 | 2 |
| 19.17 | 100 |
| 19.59 | 13 |
| 19.90 | 3 |
| 20.74 | 20 |
| 20.81 | 19 |
| 21.26 | 24 |
| 21.52 | 5 |
| 22.71 | 58 |
| 23.25 | 7 |
| 24.01 | 13 |
| 25.07 | 5 |
| 25.61 | 7 |
| 25.80 | 4 |
| 26.27 | 6 |
| 26.89 | 5 |
| 27.26 | 1 |
| 28.13 | 5 |
| 28.71 | 1 |
| 29.17 | 3 |
| 29.41 | 2 |
| 30.17 | 5 |
| 30.66 | 6 |
| 31.99 | 3 |
| 32.39 | 1 |
| 36.90 | 1 |
| 39.49 | 3 |

This cocrystal improves greatly the solubility of bilastine as shown in Figure 4.2.d).

### Example 5. Bilastine/ benzoic acid cocrystals

### Example 5.1. Crystalline form BE(I)_bilastine/ benzoic acid 1:2 cocrystal

The crystalline form was obtained by wet grinding a 1:2 mixture of bilastine: benzoic acid in ACN, MeOH, EtOH, IPA, EtOAc, THF, TBME, DCM, Toluene, DMF and NMP.

This crystalline form was also obtained by slurrying bilastine and benzoic acid at a stoichiometry ratio of 1:2 in water, toluene or TBME.

This crystalline form was also obtained by seeding a suspension of BLN(I) (1g, 2.16 mmol) and benzoic acid (527 mg, 4.314 mmol in water (10 ml) with BE(I). The resulting suspension was stirred for 18 hours at rt. The obtained solid was isolated by filtration, washed with water (2 x 1 mL) and dried under vacuum, to yield 1.48 g of BE(I) as a white solid (yield 97%).

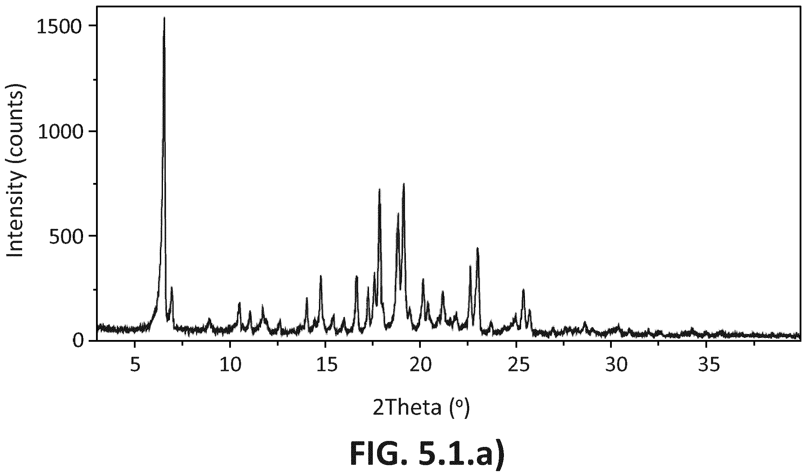

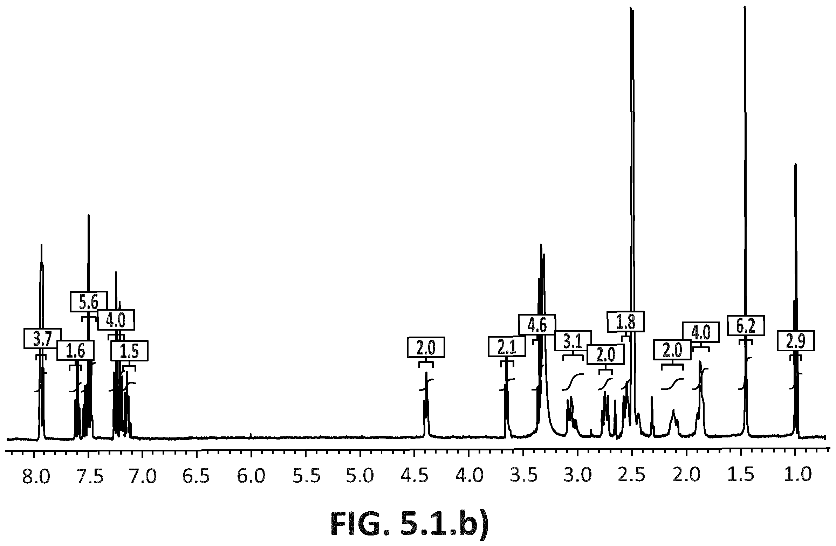

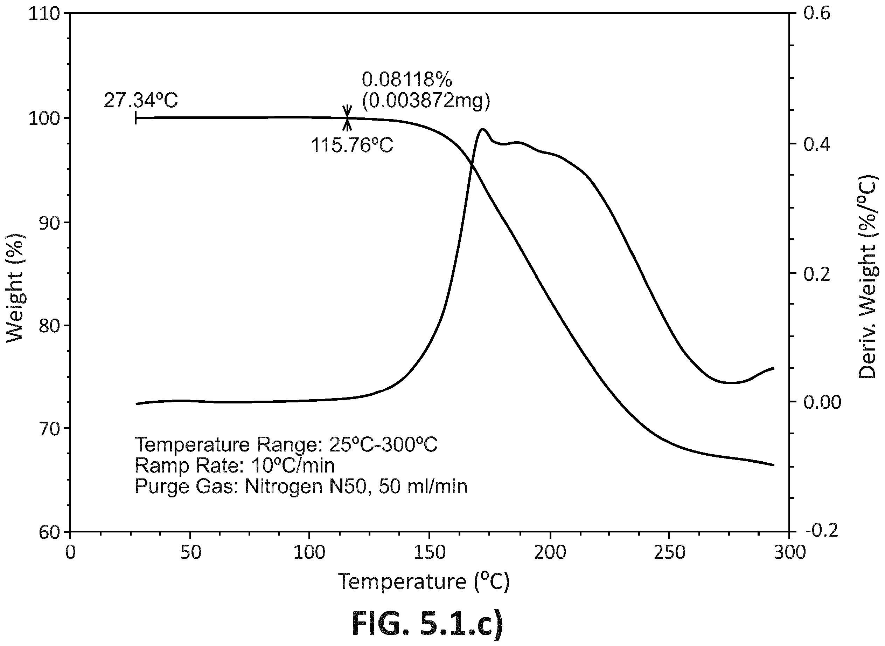

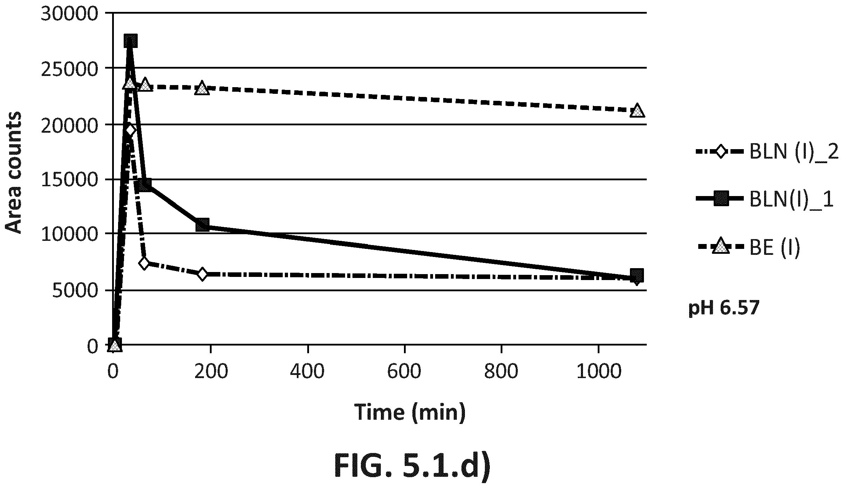

The resulting cocrystal was characterised by XPRD (see Fig. 5.1, a)) and ¹H-NMR (see Fig. 5.1, b)) and TGA (Fig. 5.1, c)). XPDR analysis confirmed that this form was stable forced conditions of humidity and temperature (75 ± 5% RH, 40 °C) for at least 1 week. A 1:2 ratio of bilastine:benzoic acid was confirmed by ¹H-NMR.

**Table 5.1. XRPD peak list of BE(I):**

| Pos. [°2Th.] | Rel. Int. [%] |
|---|---|
| 6.62 | 100 |
| 7.01 | 13 |
| 8.98 | 4 |
| 10.56 | 10 |
| 11.10 | 7 |
| 11.78 | 8 |
| 12.69 | 5 |
| 14.08 | 10 |
| 14.80 | 20 |
| 15.45 | 5 |
| 16.04 | 5 |
| 16.67 | 19 |
| 17.27 | 14 |
| 17.61 | 17 |
| 17.88 | 48 |
| 18.08 | 7 |
| 18.89 | 36 |
| 19.18 | 51 |
| 19.51 | 6 |
| 20.19 | 18 |
| 20.44 | 8 |
| 21.23 | 11 |
| 21.94 | 4 |
| 22.65 | 23 |
| 23.02 | 30 |
| 23.76 | 3 |
| 25.01 | 5 |
| 25.44 | 15 |
| 25.73 | 8 |
| 26.99 | 2 |
| 27.64 | 2 |
| 28.67 | 3 |
| 30.41 | 2 |
| 30.94 | 2 |
| 31.99 | 1 |

This cocrystal improves greatly the solubility of bilastine as shown in Figure 5.1.d).

### Example 5.2. Crystalline form BE(II)_bilastine/ benzoic acid 2:1 cocrystal

The crystalline form was obtained by slurrying bilastine and benzoic acid at a stoichiometry ratio of 2:1 in water. After stirring for 15 h, the suspension was filtered and the solid washed with water and dried under vacuum.

This crystalline form was also obtained by adding benzoic acid (132 mg, 1.078 mmol) to a suspension of BLN(I) (1g, 2.16 mmol) in water (10 ml). The mixture was seeded with BE(II) and stirred for 18 hours at rt. The obtained solid was isolated by filtration, washed with water (2 x 1 mL) and dried under vacuum, to yield 1.104 g of BE(II) as a white solid (yield 92%).

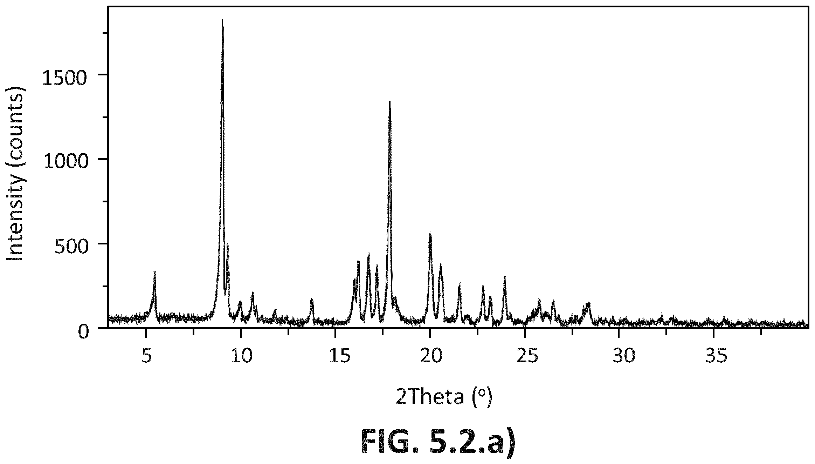

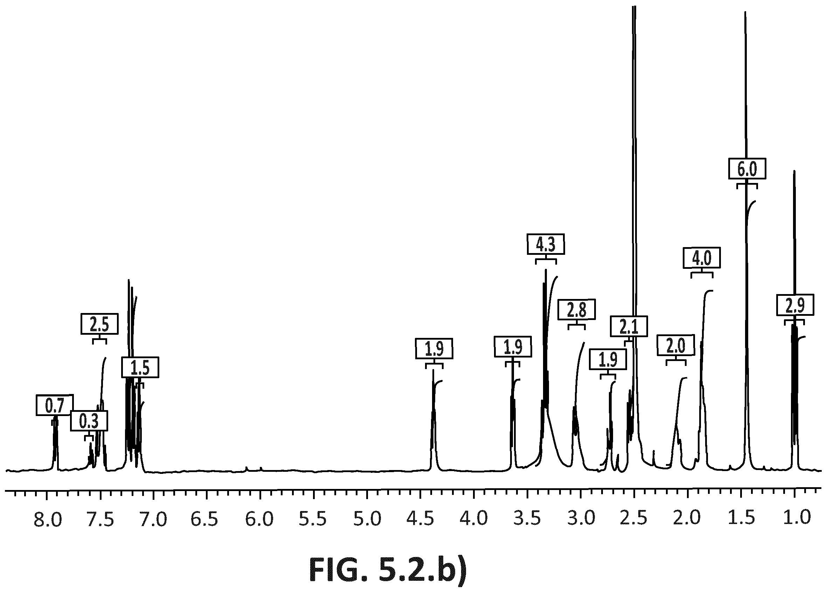

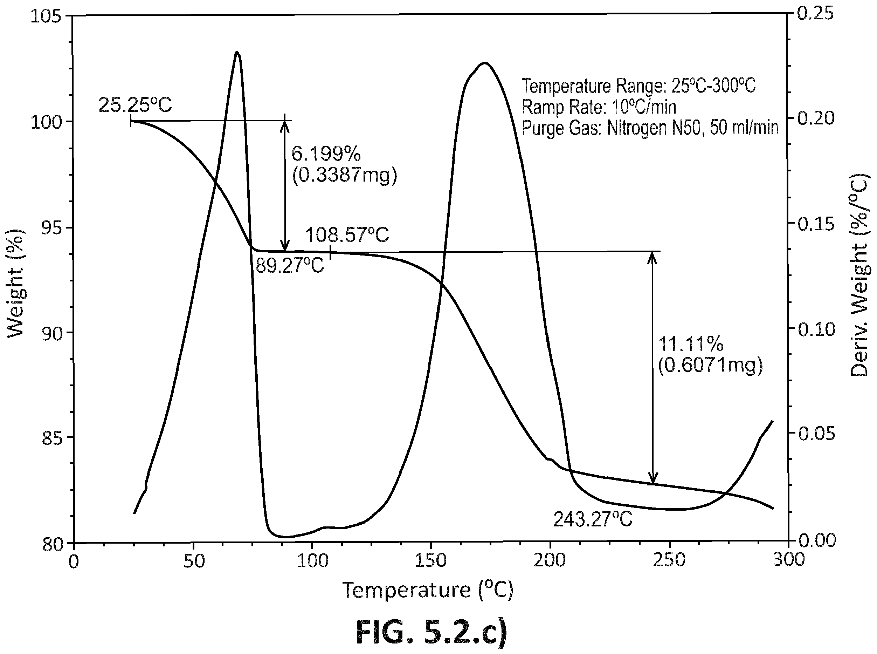

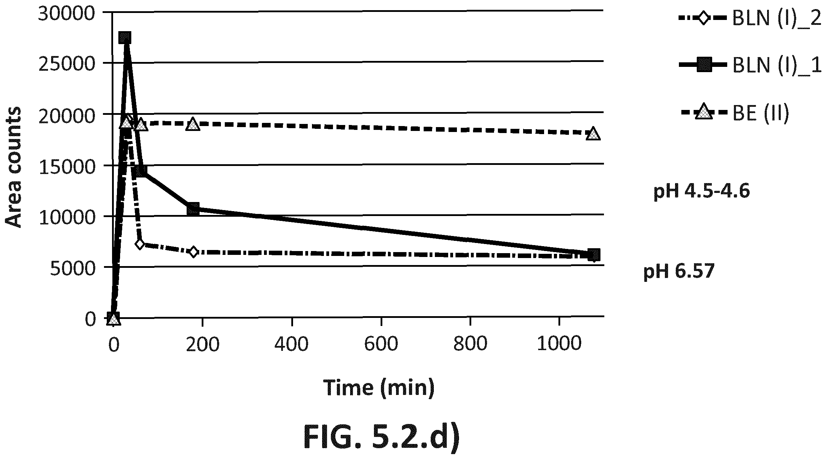

The resulting cocrystal was characterised by XPRD (see Fig. 5.2, a)), ¹H-NMR (see Fig. 5.2, b)) and TGA (see Fig. 5.2, c)). XPDR analysis confirmed that this form was stable forced conditions of humidity and temperature (75 ± 5% RH, 40 °C) for at least 1 week. A 2:1 ratio of bilastine:benzoic acid was confirmed by ¹H-NMR.

**Table 5.2. XRPD peak list of BE(II):**

| Pos. [°2Th.] | Rel. Int. [%] |
|---|---|
| 5.58 | 16 |
| 6.55 | 1 |
| 9.15 | 100 |
| 9.42 | 23 |
| 10.08 | 5 |
| 10.72 | 9 |
| 10.90 | 4 |
| 11.89 | 3 |
| 12.50 | 2 |
| 13.87 | 7 |
| 16.04 | 14 |
| 16.29 | 19 |
| 16.80 | 21 |
| 17.27 | 19 |
| 17.94 | 74 |
| 18.21 | 7 |
| 20.07 | 30 |
| 20.55 | 16 |
| 20.70 | 14 |
| 21.60 | 12 |
| 21.98 | 2 |
| 22.82 | 13 |
| 23.21 | 9 |
| 23.98 | 15 |
| 24.30 | 3 |
| 25.42 | 3 |
| 25.79 | 8 |
| 26.13 | 3 |
| 26.53 | 7 |
| 26.79 | 2 |
| 27.43 | 2 |
| 27.76 | 2 |
| 28.12 | 4 |
| 28.41 | 6 |
| 28.98 | 1 |
| 32.16 | 2 |
| 32.68 | 2 |
| 34.67 | 1 |
| 35.52 | 1 |

This cocrystal improves greatly the solubility of bilastine as shown in Figure 5.2.d).

### Example 6. Bilastine/ 4-aminobenzoic acid cocrystals

### Example 6.1. Crystalline form AB(VII)_bilastine/ 4-aminobenzoic acid 1:1 cocrystal

The crystalline form was obtained by slurrying a mixture of bilastine and 4-aminobenzoic acid at a stoichiometry ratio of 1:2 in ACN. After stirring for 15 h, the suspension was filtered and the solid washed with ACN and dried under vacuum.

This crystalline form was also obtained by adding 4-aminobenzoic acid (592 mg, 4.314 mmol) to a suspension of BLN(I) (1000 mg, 2.157 mmol) in ACN (10 ml). The resulting suspension was seeded with AB(VII) and stirred for 18 hours at rt. The obtained solid was isolated by filtration, washed with acetonitrile (2 x 1 mL) and dried under vacuum, to yield 1.276 g of AB(VII) as a white solid (yield 77%).

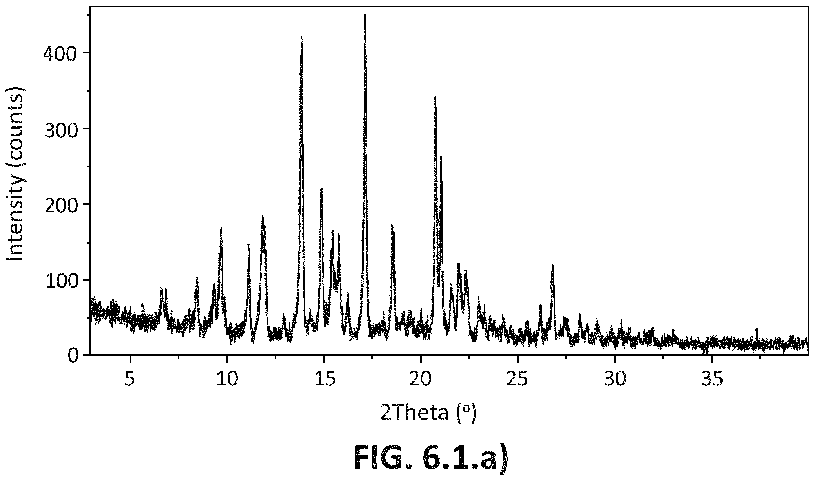

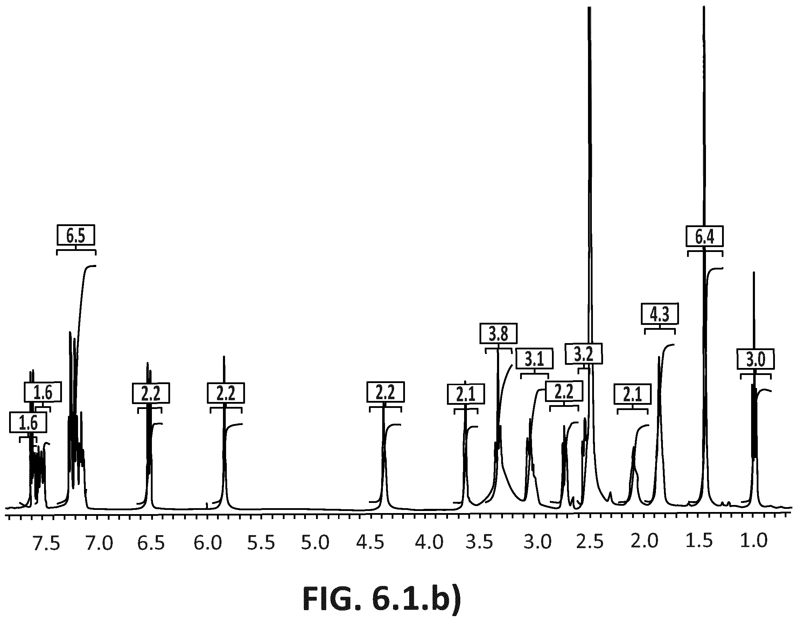

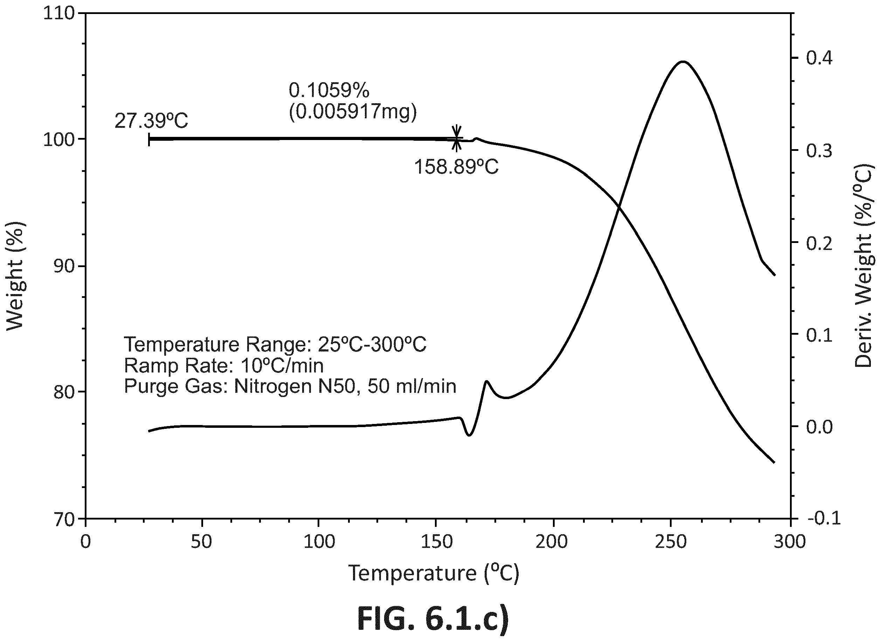

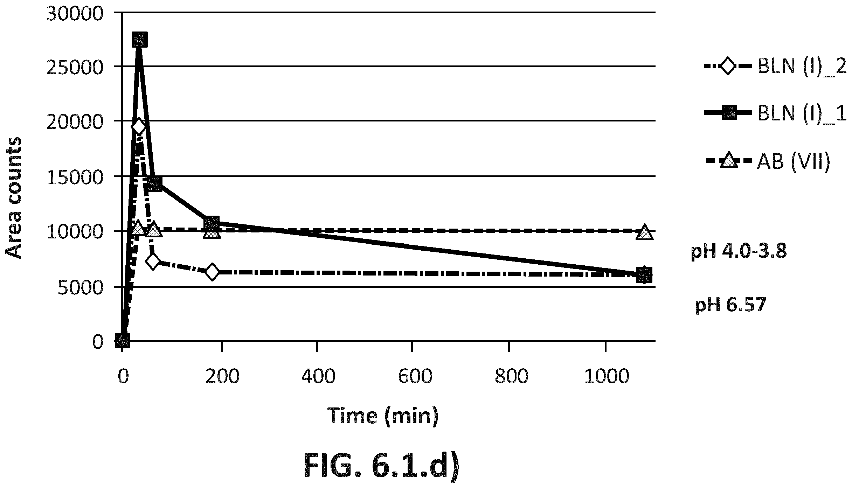

The resulting cocrystal was characterised by XPRD (see Fig. 6.1, a)) and ¹H-NMR (see Fig. 6.1, b)) and TGA (Fig. 6.1, c)). XPDR analysis confirmed that this form was stable forced conditions of humidity and temperature (75 ± 5% RH, 40 °C) for at least 1 week. A 1:1 ratio of bilastine: 4-aminobenzoic acid was confirmed by ¹H-NMR.

**Table 6.1. XRPD peak list of AB(VII):**

| Pos. [°2Th.] | Rel. Int. [%] |
|---|---|
| 5.76 | 3 |
| 6.72 | 12 |
| 6.94 | 9 |
| 8.55 | 12 |
| 9.43 | 15 |
| 9.80 | 25 |
| 11.19 | 26 |
| 11.87 | 35 |
| 12.03 | 28 |
| 12.99 | 6 |
| 13.91 | 93 |
| 14.94 | 43 |
| 15.48 | 30 |
| 15.84 | 27 |
| 16.31 | 10 |
| 17.19 | 100 |
| 18.59 | 35 |
| 19.16 | 6 |
| 19.46 | 5 |
| 20.02 | 4 |
| 20.78 | 72 |
| 21.06 | 53 |
| 21.57 | 13 |
| 21.95 | 22 |
| 22.31 | 18 |
| 23.01 | 10 |
| 24.28 | 5 |
| 25.47 | 5 |
| 26.17 | 8 |
| 26.78 | 24 |
| 27.43 | 5 |
| 28.20 | 6 |
| 28.56 | 4 |
| 29.07 | 4 |
| 30.26 | 2 |
| 31.93 | 2 |
| 32.96 | 1 |
| 39.04 | 2 |

This cocrystal improves the solubility of bilastine as shown in Figure 6.1.d).

### Example 6.2. Crystalline form AB(VIII)_bilastine/ 4-aminobenzoic acid 2:1 cocrystal

The crystalline form was obtained by slurrying a mixture of bilastine (120 mg) and 4-aminobenzoic acid at a stoichiometry ratio of 2:1 in water (15 mL). After stirring for 15 h, the suspension was filtered and the solid washed with water and dried under vacuum.

This crystalline form was also obtained by adding 4-aminobenzoic acid (148 mg, 1.078 mmol) to a suspension of BLN(I) (1000 mg, 2.157 mmol) in water (10 ml). The resulting suspension was seeded with AB(VIII) and stirred for 18 hours at rt. The obtained solid was isolated by filtration, washed with water (2 x 1 mL) and dried under vacuum, to yield 1.143 g of AB(VIII) as a white solid (yield 95%).

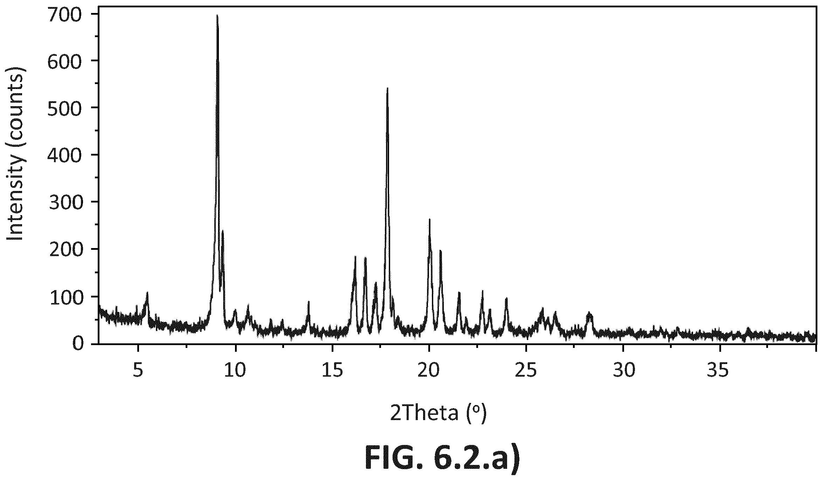

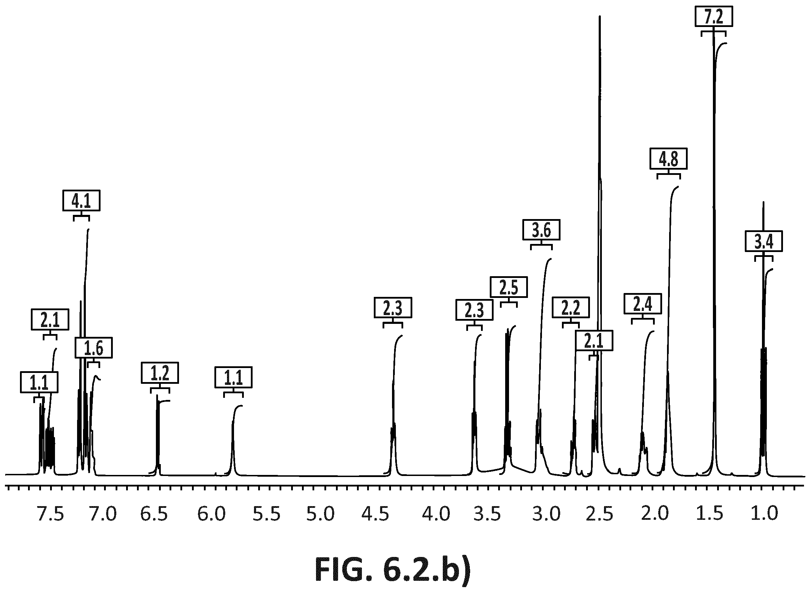

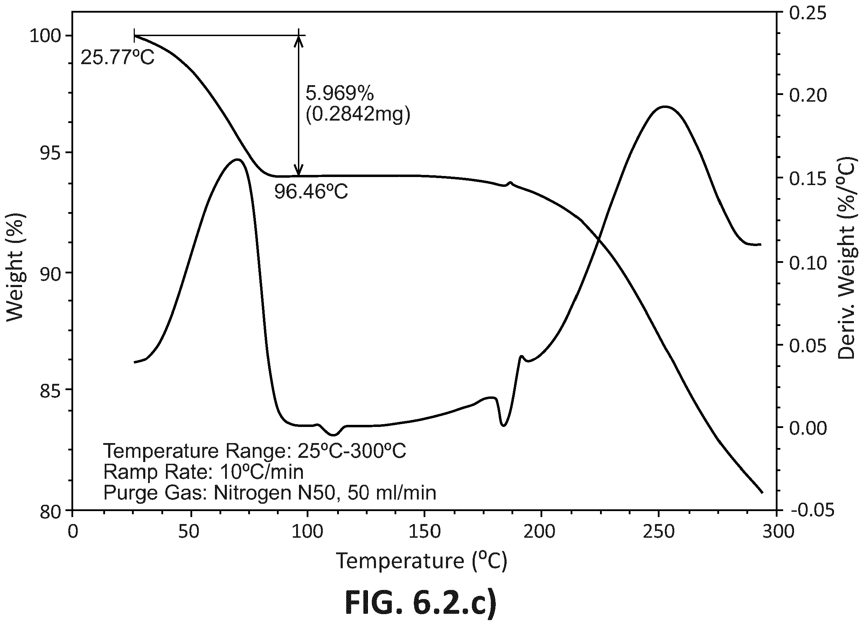

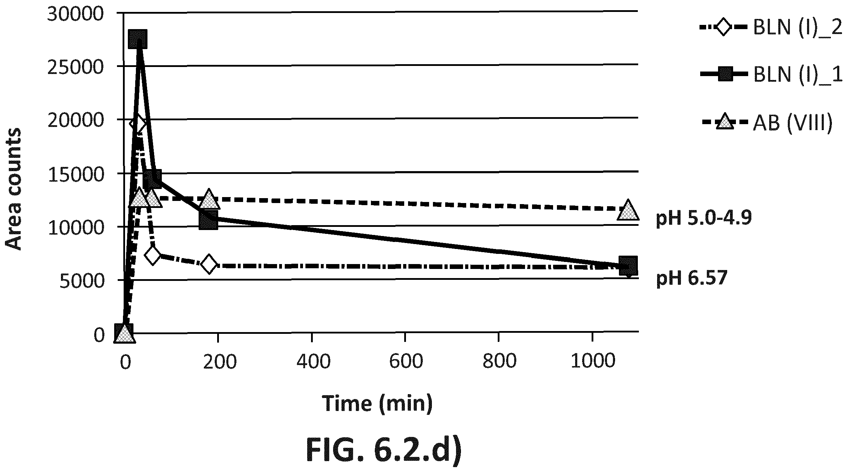

The resulting cocrystal was characterised by XPRD (see Fig. 6.2, a)), ¹H-NMR (see Fig. 6.2, b)) and TGA (see Fig. 6.2.c)). XPDR analysis confirmed that this form was stable forced conditions of humidity and temperature (75 ± 5% RH, 40 °C) for at least 1 week. A 2:1 ratio of bilastine: 4-aminobenzoic acid was confirmed by ¹H-NMR.

**Table 6.2. XRPD peak list of AB(VIII):**

| Pos. [°2Th.] | Rel. Int. [%] |
|---|---|
| 5.54 | 9 |
| 9.17 | 100 |
| 9.42 | 30 |
| 10.07 | 4 |
| 10.70 | 5 |
| 11.88 | 2 |
| 12.46 | 3 |
| 13.81 | 10 |
| 16.23 | 15 |
| 16.79 | 20 |
| 17.28 | 14 |
| 17.88 | 79 |
| 18.17 | 9 |
| 18.43 | 3 |
| 20.06 | 36 |
| 20.63 | 25 |
| 21.56 | 12 |
| 21.92 | 4 |
| 22.77 | 13 |
| 23.16 | 8 |
| 23.99 | 11 |
| 25.81 | 7 |
| 26.13 | 4 |
| 26.46 | 5 |
| 27.53 | 1 |
| 28.15 | 6 |
| 30.29 | 1 |
| 31.92 | 2 |
| 32.78 | 2 |

This cocrystal improves the solubility of bilastine as shown in Figure 6.2.d).

### Example 7. Bilastine/ L-malic acid cocrystals

### Example 7.1. Crystalline form L-ML(I)_bilastine/ L-malic acid 2:1 cocrystal

The crystalline form was obtained by slurrying or by wet-grinding a mixture of bilastine and L-malic acid at a stoichiometry ratio of 1:2 in water.

This crystalline form was also obtained by adding L-malic acid (578.5 mg, 4.314 mmol) to a suspension of BLN(I) (1000 mg, 2.157 mmol) in water (10 ml). The resulting suspension was seeded with L-ML(I) and stirred for 18 hours at rt. The obtained solid was isolated by filtration, washed with water (2 x 1 mL) and dried under vacuum, to yield 985 mg of L-ML(I) as a white solid (yield 82%).

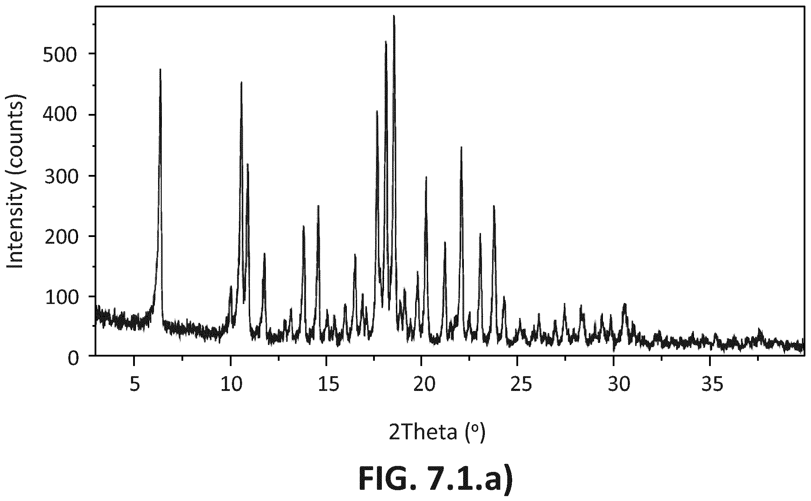

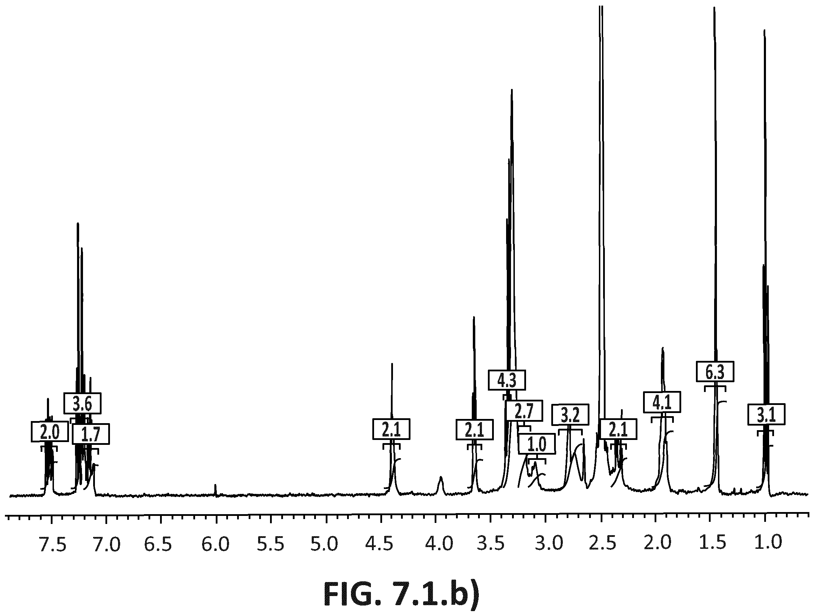

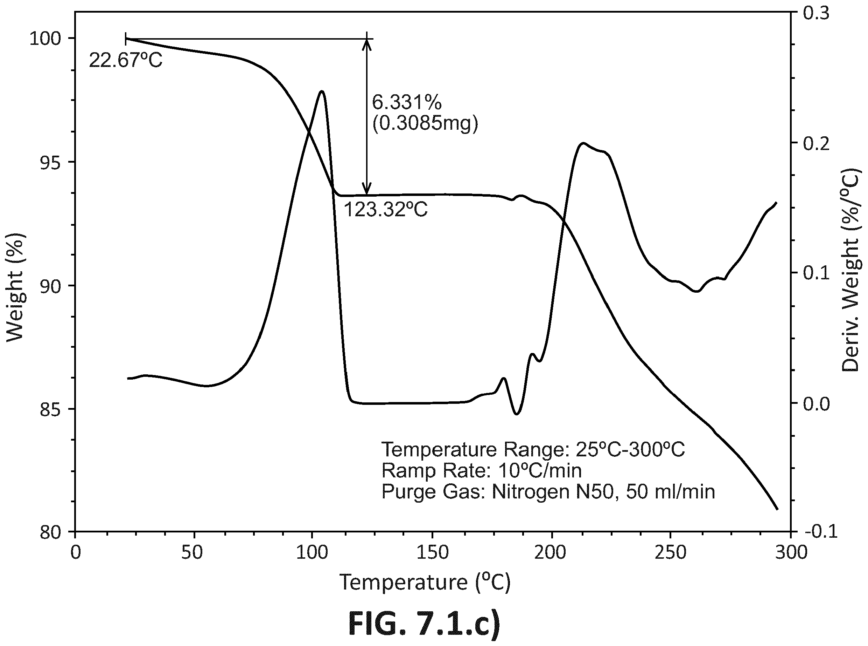

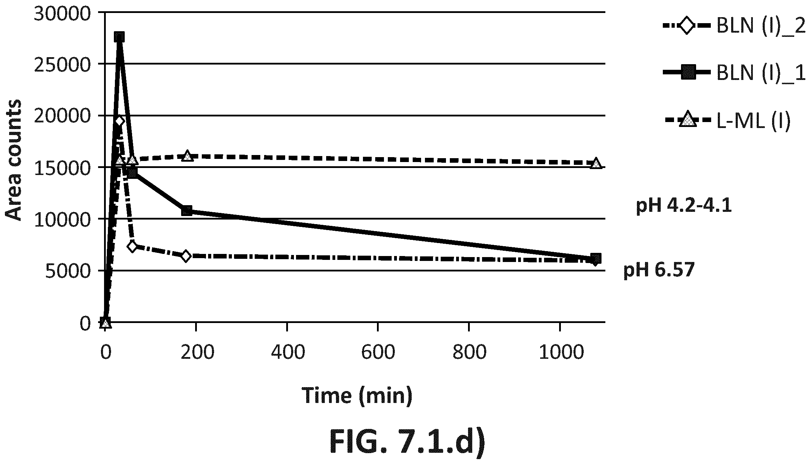

The resulting cocrystal was characterised by XPRD (see Fig. 7.1, a)), ¹H-NMR (see Fig. 7.1, b)) and TGA (see Fig. 7.1, c)). XPDR analysis confirmed that this form was stable forced conditions of humidity and temperature (75 ± 5% RH, 40 °C) for at least 1 week. A 2:1 ratio of bilastine:L-malic acid was confirmed by ¹H-NMR.

**Table 7.1. XRPD peak list of L-ML(I):**

| Pos. [°2Th.] | Rel. Int. [%] |
|---|---|
| 6.43 | 73 |
| 10.06 | 12 |
| 10.64 | 71 |
| 10.96 | 48 |
| 11.82 | 26 |
| 12.87 | 5 |
| 13.18 | 9 |
| 13.89 | 29 |
| 14.61 | 39 |
| 15.09 | 8 |
| 15.45 | 7 |
| 16.01 | 10 |
| 16.55 | 25 |
| 16.93 | 11 |
| 17.14 | 6 |
| 17.69 | 70 |
| 18.14 | 92 |
| 18.56 | 100 |
| 18.87 | 8 |
| 19.12 | 13 |
| 19.41 | 4 |
| 19.80 | 18 |
| 20.21 | 45 |

| 21.22 | 28 |
|---|---|
| 21.51 | 2 |
| 22.04 | 56 |
| 22.48 | 7 |
| 23.03 | 32 |
| 23.76 | 41 |
| 24.30 | 12 |
| 25.10 | 5 |
| 25.81 | 5 |
| 26.09 | 8 |
| 26.38 | 2 |
| 26.92 | 6 |
| 27.39 | 10 |
| 27.91 | 4 |
| 28.24 | 11 |
| 29.34 | 7 |
| 29.81 | 7 |
| 30.54 | 10 |
| 31.02 | 4 |
| 32.22 | 2 |
| 34.06 | 3 |
| 35.23 | 4 |
| 37.55 | 3 |
| 38.46 | 2 |

This cocrystal improves greatly the solubility of bilastine as shown in Figure 7.1.d).

### Example 8. Bilastine/ resorcinol cocrystals

### Example 8.1. Crystalline form RE(IV)_bilastine/ resorcinol acid 2:1 cocrystal

The crystalline form was obtained by slurrying a mixture of bilastine and resorcinol at a stoichiometry ratio of 1:2 in acetone and at a stoichiometry ratio of 2:1 in chlorophorm.

The crystalline form was also obtained by slurrying a mixture of bilastine and resorcinol at a stoichiometry ratio of 1:1 or 1:2 in acetone. After stirring for 15 h, the suspension was filtered and the solid washed with acetone and dried under vacuum.

This crystalline form was also obtained by adding resorcinol (119 mg, 1.078 mmol) to a suspension of BLN(I) (1000 mg, 2.157 mmol) in AcOEt (10 ml). The resulting suspension was seeded with RE(IV) and stirred for 18 hours at rt. The obtained solid was isolated by filtration, washed with AcOEt (2 x 1 mL) and dried under vacuum, to yield 1.099 mg of RE(IV) as a white solid (yield 98%).

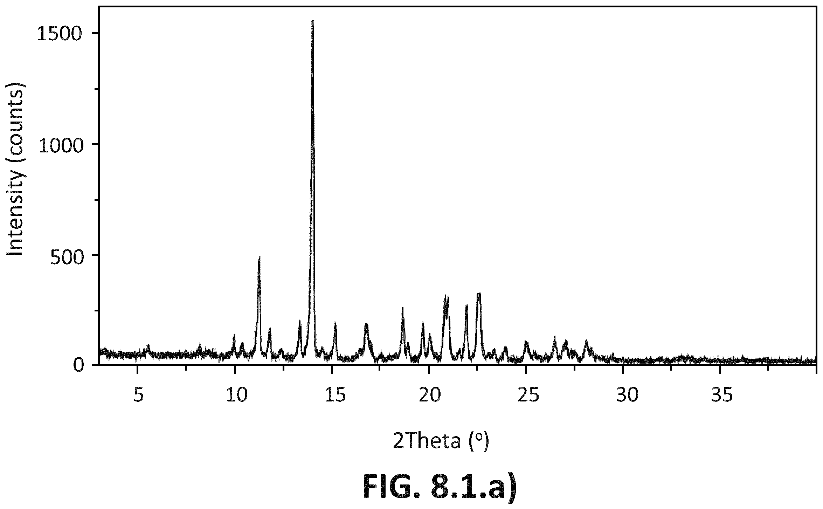

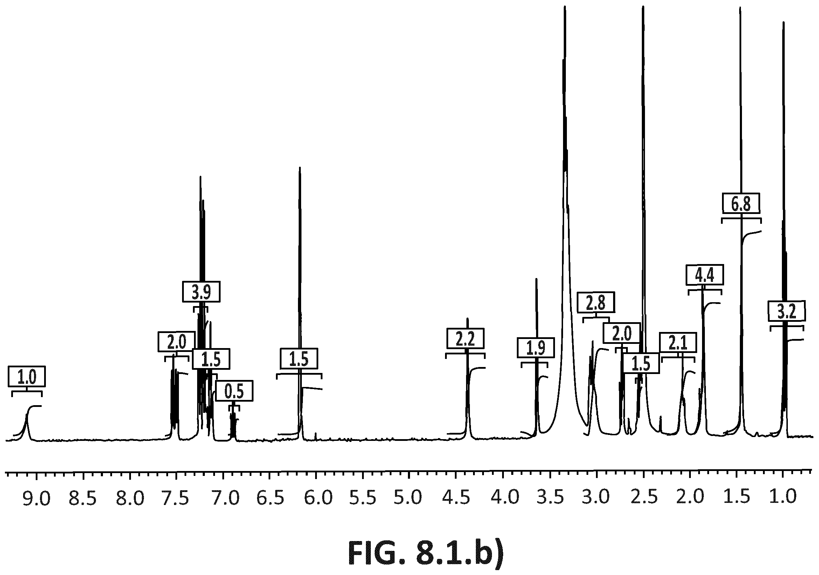

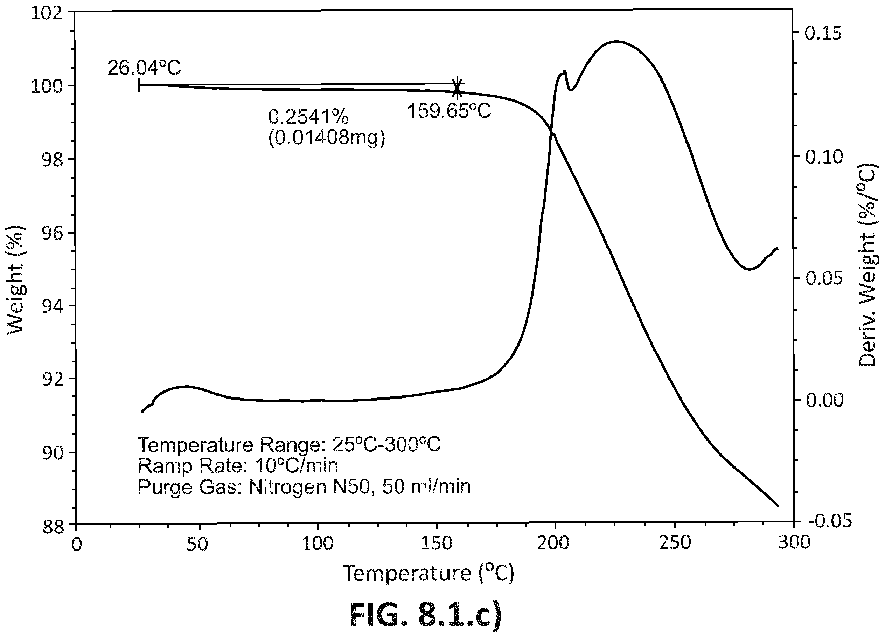

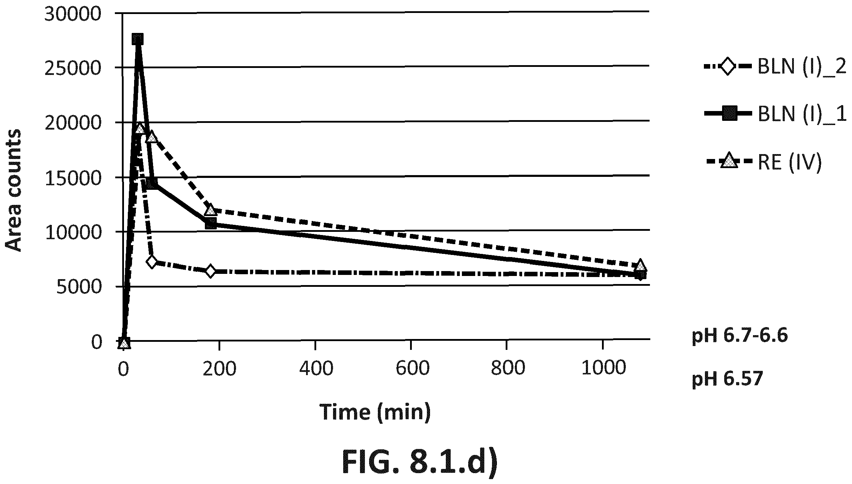

The resulting cocrystal was characterised by XPRD (see Fig. 8.1, a)), ¹H-NMR (see Fig. 8.1, b)) and TGA (Fig. 8.1, c)). XPDR analysis confirmed that this form was stable forced conditions of humidity and temperature (75 ± 5% RH, 40 °C) for at least 1 week. A 2:1 ratio of bilastine: resorcinol was confirmed by ¹H-NMR.

**Table 8.2. XRPD peak list of RE(IV):**

| Pos. [°2Th.] | Rel. Int. [%] |
|---|---|
| 5.59 | 3 |
| 8.24 | 2 |
| 8.71 | 1 |
| 10.00 | 5 |
| 10.42 | 4 |
| 11.30 | 29 |
| 11.85 | 7 |
| 12.44 | 2 |
| 13.37 | 9 |
| 14.03 | 100 |
| 14.54 | 2 |
| 15.20 | 10 |
| 16.74 | 10 |
| 17.01 | 4 |
| 18.68 | 13 |
| 18.96 | 4 |
| 19.70 | 9 |
| 20.05 | 6 |
| 20.82 | 17 |
| 21.01 | 18 |
| 21.57 | 2 |
| 21.96 | 13 |
| 22.52 | 19 |
| 22.64 | 19 |
| 23.37 | 3 |
| 23.89 | 3 |
| 25.01 | 5 |
| 26.45 | 5 |
| 27.07 | 4 |
| 27.60 | 1 |
| 28.09 | 6 |
| 28.35 | 3 |

This cocrystal improves greatly the solubility of bilastine as shown in Figure 8.1.d).

### Example 9. Bilastine/ methyl 4-hydroxybenzoate cocrystals

### Example 9.1. Crystalline form bilastine/ methyl 4-hydroxybenzoate 2:1 cocrystal

The crystalline form was obtained by the following procedure: Bilastine (500 mg) and methyl 4-hydroxybenzoate (1 eq, 83 mg) were suspended in water (8 ml) at 65°C. The obtained solid was filtered off, washed with ethanol and dried under vacuum. (Yield 480 mg - 83%).

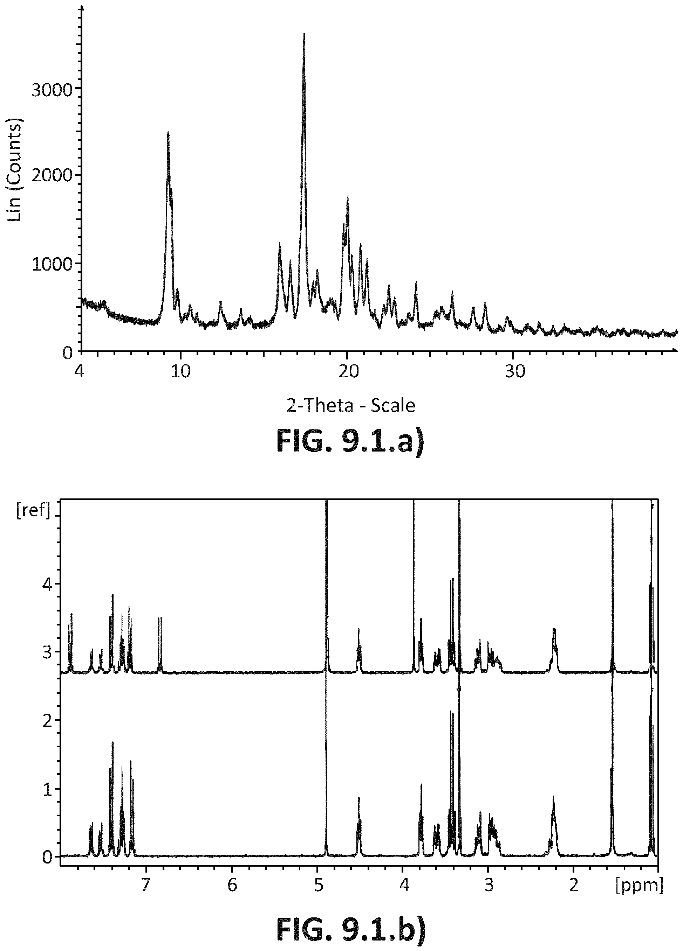

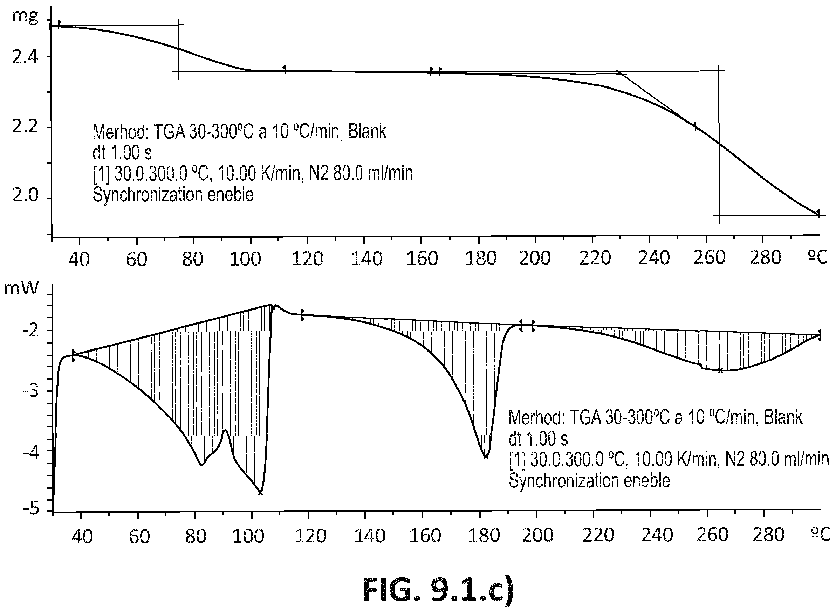

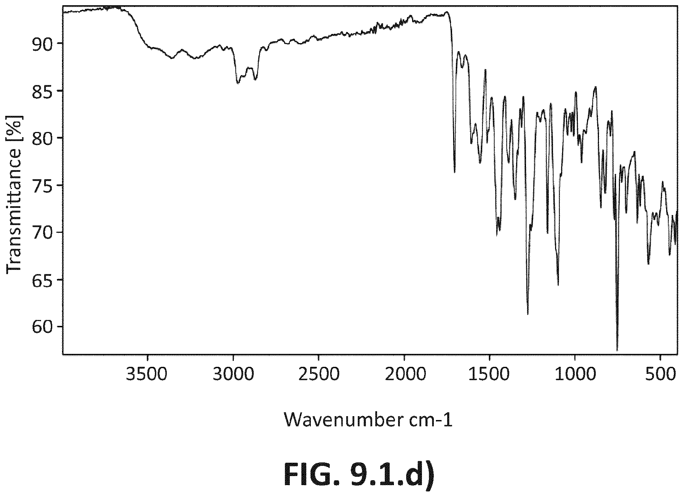

The resulting cocrystal was characterised by XPRD (see Fig. 9.1, a)), ¹H-NMR (see Fig. 9.1, b)), TGA (see Fig. 9.1, c)) and FT-IR (see Fig. 9.1 d)). A 1:2 ratio of bilastine:methyl 4-hydroxybenzoate was confirmed by ¹H-NMR. The cocrystal was found to be stable for at least 4 h after heating at 2-3 mbar and 40°C.

### Example 10. Bilastine/ N-4-hydroxyphenylacetamide cocrystals

### Example 10.1. Crystalline form bilastine/ N-4-hydroxyphenylacetamide cocrystal

The cocrystal was obtained via slurry in water.

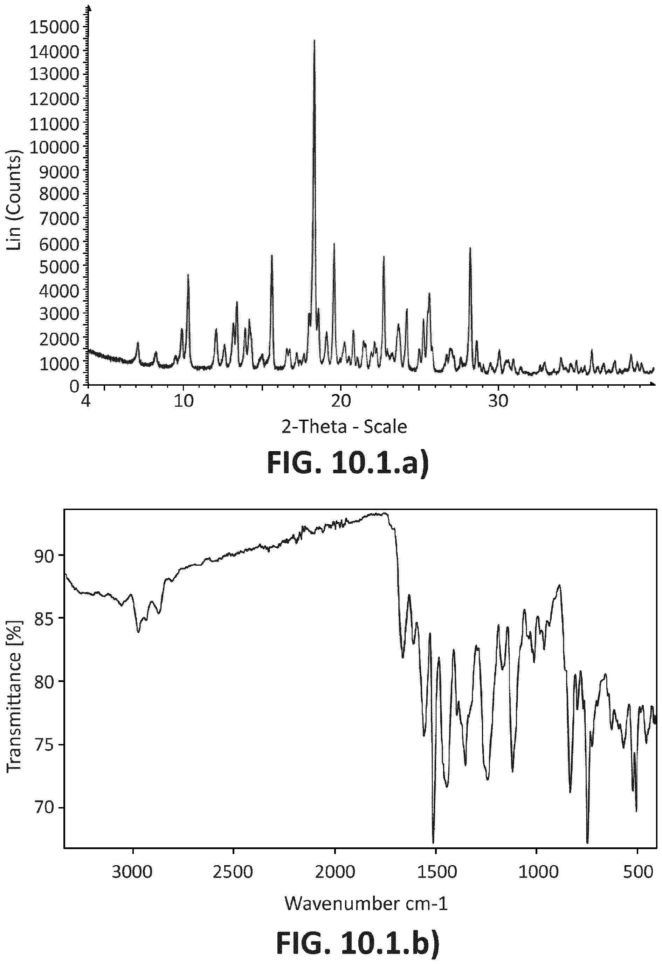

The resulting cocrystal was characterised by XPRD (see Fig. 10.1, a)) and FT-IR (see Fig. 10.1, b)).

## Claims

1. A cocrystal comprising
a) at least bilastine; and
b) at least a cocrystal forming compound selected from the group consisting of glutaric acid, adipic acid, sorbic acid, succinic acid, benzoic acid, 4-aminobenzoic acid, L-malic acid, resorcinol, methyl 4-hydroxy benzoate and N-(4-hydroxyphenyl)acetamide and mixtures thereof, or a solvate thereof.

2. Cocrystal according to claim 1 selected from the group consisting of a cocrystal of bilastine and glutaric acid in a 2:1 molar ratio, a cocrystal of bilastine and glutaric acid in a 1:1 molar ratio, a cocrystal of bilastine and adipic acid in a 1:1 molar ratio, a cocrystal of bilastine and adipic acid in a 2:1 molar ratio, a cocrystal of bilastine and sorbic acid in a 2:1 molar ratio, a cocrystal of bilastine and succinic acid in a 1:1 molar ratio, a cocrystal of bilastine and succinic acid in a 2:1 molar ratio, a cocrystal of bilastine and benzoic acid in a 1:2 molar ratio, a cocrystal of bilastine and benzoic acid in a 2:1 molar ratio, a cocrystal of bilastine and 4-aminobenzoic acid in a 1:1 molar ratio, a cocrystal of bilastine and 4-aminobenzoic acid in a 2:1 molar ratio, a cocrystal of bilastine and L-malic in a 2:1 molar ratio, a cocrystal of bilastine and resorcinol in a 2:3 molar ratio, a cocrystal of bilastine and resorcinol in a 2:1 molar ratio and a cocrystal of bilastine and methyl 4-hydroxybenzoate in a 2:1 molar ratio,
or a hydrate thereof.

3. Cocrystal according to claim 2 selected from the group consisting of a cocrystal of bilastine and glutaric acid in a 2:1 molar ratio and a cocrystal of bilastine and glutaric acid in a 1:1 molar ratio, or a hydrate thereof.

4. A process for preparing a cocrystal as defined in any one of claims 1 to 3 comprising:
a) stirring a mixture of bilastine, or a pharmaceutically acceptable solvate or polymorph thereof, and the neutral cocrystal forming compound in an appropriate solvent between room temperature and 40°C;
b) cooling the mixture to room temperature if temperature of step a) is higher than room temperature, and
c) isolating the obtained compound.

5. A process for preparing a cocrystal as defined in any one of claims 1 to 3 comprising:
a) wet grinding of bilastine, or a pharmaceutically acceptable solvate or polymorph thereof, and the neutral cocrystal forming compound in an appropriate solvent, and
b) isolating the obtained compound.

6. Process according to anyone of claims 4 or 5, wherein the appropriate solvent is selected from water, acetonitrile, dimethylsulfoxide, methanol, ethanol, isopropyl alcohol, ethyl acetate, isobutyl acetate, acetone, methyl isobutyl ketone, tetrahydrofurane, dioxane, diethylether, methyl tert-butyl ether, dichloromethane, chloroform, toluene, cyclohexane, xylene, heptane, dimethylformamide and N-methyl-2-pyrrolidone.

7. A pharmaceutical composition comprising a cocrystal as defined in any one of claims 1 to 3 and a pharmaceutically acceptable excipient.

8. A cocrystal as defined in any one of claims 1 to 3 for use as a medicament.

9. A cocrystal as defined in any one of claims 1 to 3 for use in the prevention and/or treatment of an allergic disease or disorder.

10. Cocrystal for use according to claim 9 wherein the allergic disease or disorder is selected from rhinitis, conjunctivitis, rhinoconjunctivitis, dermatitis, urticaria and asthma.

## Patentansprüche

1. Ein Co-Kristall, umfassend
a) mindestens Bilastin; und
b) mindestens eine Co-Kristall bildende Verbindung, ausgewählt aus der Gruppe bestehend aus Glutarsäure, Adipinsäure, Sorbinsäure, Bernsteinsäure, Benzoesäure, 4-Aminobenzoesäure, L-Apfelsäure, Resorcinol, Methyl-4-hydroxybenzoat und N-(4-Hydroxyphenyl)acetamid und Gemischen davon, oder ein Solvat davon.

2. Co-Kristall nach Anspruch 1, ausgewählt aus der Gruppe bestehend aus einem Co-Kristall von Bilastin und Glutarsäure in einem Molverhältnis von 2:1, einem Co-Kristall von Bilastin und Glutarsäure in einem Molverhältnis von 1:1, einem Co-Kristal von Bilastin und Adipinsäure in einem Molverhältnis von 1:1, einem Co-Kristall von Bilastin und Adipinsäure in einem Molverhältnis von 2:1, einem Co-Kristall von Bilastin und Sorbinsäure in einem Molverhältnis von 2:1, einem Co-Kristall von Bilastin und Bernsteinsäure in einem Molverhältnis von 1:1, einem Co-Kristall von Bilastin und Bernsteinsäure in einem Molverhältnis von 2:1, einem Co-Kristall von Bilastin und Benzoesäure in einem Molverhältnis von 1:2, einem Co-Kristall von Bilastin und Benzoesäure in einem Molverhältnis von 2:1, einem Co-Kristall von Bilastin und 4-Aminobenzoesäure in einem Molverhältnis von 1:1, einem Co-Kristall von Bilastin und 4-Aminobenzoesäure in einem Molverhältnis von 2:1, einem Co-Kristall von Bilastin und L-Apfelsäure in einem Molverhältnis von 2:1, einem Co-Kristall von Bilastin und Resorcinol in einem Molverhältnis von 2:3, einem Co-Kristall von Bilastin und Resorcinol in einem Molverhältnis von 2:1 und einem Co-Kristall von Bilastin und Methyl-4-hydroxybenzoat in einem Molverhältnis von 2:1,
oder ein Hydrat davon.

3. Co-Kristall nach Anspruch 2, ausgewählt aus der Gruppe bestehend aus einem Co-Kristall von Bilastin und Glutarsäure in einem Molverhältnis von 2:1 und einem Co-Kristall von Bilastin und Glutarsäure in einem Molverhältnis von 1:1, oder ein Hydrat davon.

4. Ein Verfahren zur Herstellung eines Co-Kristalls wie in einem der Ansprüche 1 bis 3 definiert, umfassend:
a) Rühren eines Gemischs aus Bilastin oder einem pharmazeutisch verträglichen Solvat oder Polymorph davon, und der neutralen Co-Kristall bildenden Verbindung in einem geeigneten Lösungsmittel zwischen Raumtemperatur und 40°C;
b) Abkühlen des Gemischs auf Raumtemperatur, wenn die Temperatur von Schritt a) höher als die Raumtemperatur ist, und
c) Isolieren der erhaltenen Verbindung.

5. Ein Verfahren zur Herstellung eines Co-Kristalls wie in einem der Ansprüche 1 bis 3 definiert, umfassend:
a) Nassmahlen von Bilastin oder eines pharmazeutisch verträglichen Solvats oder Polymorphs davon, und der neutralen Co-Kristall bildenden Verbindung in einem geeigneten Lösungsmittel, und
b) Isolieren der erhaltenen Verbindung.

6. Verfahren nach einem der Ansprüche 4 oder 5, wobei das geeignete Lösungsmittel aus Wasser, Acetonitril, Dimethylsulfoxid, Methanol, Ethanol, Isopropylalkohol, Ethylacetat, Isobutylacetat, Aceton, Methylisobutylketon, Tetrahydrofuran, Dioxan, Diethylether, Methyl-tert-butylether, Dichlormethan, Chloroform, Toluol, Cyclohexan, Xylol, Heptan, Dimethylformamid und N-Methyl-2-pyrrolidon ausgewählt ist.

7. Ein Arzneimittel, umfassend einen Co-Kristall wie in einem der Ansprüche 1 bis 3 definiert und einen pharmazeutisch verträglichen Exzipienten.

8. Ein Co-Kristall wie in einem der Ansprüche 1 bis 3 definiert, zur Verwendung als ein Medikament.

9. Ein Co-Kristall wie in einem der Ansprüche 1 bis 3 definiert, zur Verwendung bei der Vorbeugung und/oder Behandlung einer allergischen Erkrankung oder Störung.

10. Co-Kristall zur Verwendung nach Anspruch 9, wobei die allergische Erkrankung oder Störung aus Rhinitis, Konjunktivitis, Rhinokonjunktivitis, Dermatitis, Urticaria und Asthma ausgewählt ist.

## Revendications

1. Un co-cristal comprenant
a) au moins de la bilastine ; et
b) au moins un composé formant un co-cristal choisi dans le groupe constitué par l'acide glutarique, l'acide adipique, l'acide sorbique, l'acide succinique, l'acide benzoïque, l'acide 4-aminobenzoïque, l'acide L-malique, le résorcinol, le 4-hydroxybenzoate méthylique et le N-(4-hydroxyphényl)acétamide et les mélanges de ceux-ci, ou un solvate de ceux-ci.

2. Co-cristal selon la revendication 1 choisi dans le groupe constitué d'un co-cristal de bilastine et d'acide glutarique dans un rapport molaire de 2:1, d'un co-cristal de bilastine et d'acide glutarique dans un rapport molaire de 1:1, d'un co-cristal de bilastine et d'acide adipique dans un rapport molaire de 1:1, d'un co-cristal de bilastine et d'acide adipique dans un rapport molaire de 2:1, d'un co-cristal de bilastine et d'acide sorbique dans un rapport molaire de 2:1, d'un co-cristal de bilastine et d'acide succinique dans un rapport molaire 1:1, d'un co-cristal de bilastine et d'acide succinique dans un rapport molaire de 2:1, d'un co-cristal de bilastine et d'acide benzoïque dans un rapport molaire de 1:2, d'un co-cristal de bilastine et d'acide benzoïque dans un rapport molaire de 2:1, d'un co-cristal de bilastine et d'acide 4-aminobenzoique dans un rapport molaire de 1:1, d'un co-cristal de bilastine et d'acide 4-aminobenzoique dans un rapport molaire de 2:1, d'un co-cristal de bilastine et de L-malique dans un rapport molaire de 2:1, d'un co-cristal de bilastine et de résorcinol dans un rapport molaire de 2:3, d'un co-cristal de bilastine et de résorcinol dans un rapport molaire de 2:1 et d'un co-cristal de bilastine et de méthyle 4-hydroxybenzoate dans un rapport molaire de 2:1,
ou un hydrate de ceux-ci.

3. Co-cristal selon la revendication 2 choisi dans le groupe constitué d'un co-cristal de bilastine et d'acide glutarique dans un rapport molaire de 2:1 et d'un co-cristal de bilastine et d'acide glutarique dans un rapport molaire de 1:1, ou un hydrate de ceux-ci.

4. Un procédé de préparation d'un co-cristal tel que défini dans l'une quelconque des revendications 1 à 3 comprenant :
a) le fait d'agiter un mélange de bilastine, ou d'un solvate ou d'un polymorphe pharmaceutiquement acceptable de celle-ci, et du composé neutre formant le co-cristal dans un solvant approprié entre la température ambiante et 40°C ;
b) le fait de refroidir le mélange à température ambiante si la température de l'étape a) est supérieure à la température ambiante, et
c) le fait d'isoler le composé obtenu.

5. Un procédé de préparation d'un co-cristal tel que défini dans l'une quelconque des revendications 1 à 3 comprenant :
a) le fait d'opérer un broyage humide de bilastine, ou d'un de ses solvates ou polymorphes pharmaceutiquement acceptables, et du composé neutre formant le co-cristal dans un solvant approprié, et
b) le fait d'isoler le composé obtenu.

6. Procédé selon l'une quelconque des revendications 4 ou 5, dans lequel le solvant approprié est choisi parmi l'eau, l'acétonitrile, le diméthylsulfoxyde, le méthanol, l'éthanol, l'alcool isopropylique, l'acétate d'éthyle, l'acétate d'isobutyle, l'acétone, l'acétone méthylsobutylique, le tétrahydrofuranne, le dioxanne, le diéthylether, l'éther tert-butyl méthylique, le dichlorométhane, le chloroforme, le toluène, le cyclohexanne, le xylène, le heptane, le diméthyformamide et le Nmethyl-2-pyrolidone.

7. Une composition pharmaceutique comprenant un co-cristal tel que défini dans l'une quelconque des revendications 1 à 3 et un excipient pharmaceutiquement acceptable.

8. Un co-cristal tel que défini dans l'une quelconque des revendications 1 à 3 pour une utilisation en tant que médicament.

9. Un co-cristal tel que défini dans l'une quelconque des revendications 1 à 3 pour une utilisation dans la prévention et/ou le traitement d'une maladie ou d'un trouble allergique.

10. Co-cristal destiné à être utilisé selon la revendication 9, dans lequel la maladie ou le trouble allergique est choisi parmi la rhinite, la conjonctivite, la rhinoconjonctivite, la dermatite, l'urticaire et l'asthme.
